# FASCICULE DE BREVET EUROPEEN

(11) **EP 2 547 692 B1**
(45) Date de publication et mention de la délivrance du brevet: **17.05.2017**
(21) Numéro de dépôt: 11731421.1
(22) Date de dépôt: 18.04.2011
(51) Int. Cl.: C07K 7/06, C07K 14/775, A61K 38/08, A61K 38/17, G01N 33/68, A61P 25/28

(54) **DERIVES PEPTIDIQUES, LEUR PREPARATION ET LEURS UTILISATIONS COMME VECTEURS**
PEPTIDDERIVATE, HERSTELLUNG DAVON UND VERWENDUNG DAVON ALS VEKTOREN
PEPTIDE DERIVATIVES, PREPARATION THEREOF AND USES THEREOF AS VECTORS

(30) Priorité: 21.04.2010 FR 1053036
(43) Date de publication de la demande: 23.01.2013
(73) Titulaire: Vect-Horus, 13344 Marseille Cedex 15 (FR); Centre National de la Recherche Scientifique, 75016 Paris (FR); Universite De La Mediterranee, 13284 Marseille Cedex 07 (FR)
(72) Inventeur: VLIEGHE, Patrick, F-83150 Bandol (FR); DAVID, Marion, F-13010 Marseille (FR); MOLINO, Yves, F-13620 Carry Le Rouet (FR); KHRESTCHATISKY, Michel, F-13007 Marseille (FR)
(74) Mandataire: Becker, Philippe
(86) Numéro de dépôt international: PCT/FR2011/050883
(87) Numéro de publication internationale: WO 2011/131896

(56) Documents cités:
- WO-A1-2010/046588
- WO-A2-01/81581
- WO-A2-2004/108071
- US-A1- 2004 123 343
- GOJOBORI TAKASHI: "Curated genome annotation of Oryza sativa ssp japonica and comparative genome analysis with Arabidopsis thaliana - The Rice Annotation Project", GENOME RESEARCH, [Online] vol. 17, no. 2, février 2007 (2007-02), pages 175-183, XP002615680, ISSN: 1088-9051 [extrait le 2010-12-30] & DATABASE GenBank [Online] NCBI; 2007, Itoh, T. et al: "Curative genome anotation of Oryza sativa ssp. japonica and comparative genome analysis with Arabidosis thaliana", XP002615681, Database accession no. BAF08354

## Description

L'invention concerne des dérivés peptidiques (peptides et pseudo-peptides) et leur utilisation comme vecteurs de molécules d'intérêt. L'invention concerne également des conjugués contenant un dérivé peptidique de l'invention lié à une molécule d'intérêt. Les peptides de l'invention sont utilisables notamment pour vectoriser sous forme de conjugués généralement prodrogues des molécules d'intérêt pharmaceutique ou diagnostique, comme par exemple des molécules thérapeutiques, des agents d'imagerie ou de diagnostic, ou des sondes moléculaires, à travers les membranes cellulaires, et notamment pour favoriser leur transport à travers la barrière hémato-encéphalique (BHE).

### CONTEXTE DE L'INVENTION

Selon *IMS Health,* le marché mondial des médicaments destinés à traiter les pathologies du système nerveux central (SNC, cerveau et moelle épinière) était d'environ 70 milliards de dollars en 2007, dont la part des produits issus des technologies de *drug delivery* est estimé à près de 9 milliards de dollars (Jain, 2008, Jain PharmaBiotech Report, Drug Delivery in CNS disorders)*.* Ainsi, à ce jour la neurologie fait partie des 3 aires thérapeutiques les plus importantes avec le cardiovasculaire et l'oncologie. Même si le nombre de personnes souffrant de troubles et de pathologies du SNC à travers le monde est plus important que celui des personnes atteintes par des maladies cardiovasculaires ou des cancers, la neurologie reste un marché sous-exploité. Ceci s'explique par le fait que 98% des médicaments potentiels destinés à traiter les pathologies du SNC ne traversent pas la barrière hémato-encéphalique ou BHE (Pardridge, 2003, Mol. Interv., 3, 90-105*).*

En effet, le cerveau est protégé contre les substances potentiellement toxiques par la présence de deux systèmes principaux de barrières physiologiques : la BHE, et la barrière sang-liquide céphalo-rachidien (BS-LCR). La BHE est considérée comme la principale voie pour la captation de ligands plasmatiques. Sa superficie est environ 5000 fois supérieure à celle de la BS-LCR. La longueur totale des vaisseaux sanguins constitutifs de la BHE est d'environ 600 km. Chaque cm³ de cortex cérébral contient l'équivalent d'1 km de vaisseaux sanguins. La surface totale de la BHE est estimée à 20 m² (De Boer et al., 2007, Clin. Pharmacokinet., 46 (7), 553-576)*.* Ainsi, l'endothélium cérébral, qui constitue la BHE, représente un obstacle majeur à l'utilisation de médicaments potentiels contre de nombreux troubles du SNC, mais également une surface importante d'échange potentiel entre le sang et le tissu nerveux.

En règle générale, seules quelques petites molécules lipophiles d'environ 450 à 600 Daltons peuvent passer à travers la BHE (soit seulement 2% des candidats-médicaments), c'est-à-dire, passer depuis le sang jusqu'au cerveau. Le poids moléculaire et la taille de nombreux candidats-médicaments, qui montrent des résultats prometteurs dans les études *in vitro* et chez l'animal pour le traitement des troubles du SNC, sont considérablement plus importants. Ainsi, la plupart des molécules telles que les peptides ou protéines thérapeutiques sont généralement exclues du passage/transport depuis le sang jusqu'au cerveau, en raison de la faible perméabilité transcellulaire des cellules endothéliales des capillaires cérébraux (*brain capillary endothelial cells* ou BCECs) pour ce type de candidats-médicaments. Les BCECs organisées en vaisseaux sont entourées d'une lame basale, de pieds d'astrocytes, de péricytes et de cellules microgliales et neuronales. L'association étroite des cellules endothéliales avec les pieds astrocytaires est responsable du développement et du maintien des propriétés d'imperméabilité de la BHE à la plupart des molécules, assurant ainsi un contrôle strict et efficace des échanges moléculaires entre le sang et le cerveau afin de maintenir l'homéostasie cérébrale. Les BCECs sont étroitement liées par des jonctions serrées, comparativement aux autres cellules endothéliales d'autres organes, qui sont fenestrées. Ces jonctions serrées empêchent ainsi tout transport para-cellulaire à travers la BHE.

La BHE est considérée comme l'obstacle majeur à franchir dans le développement de nouvelles thérapies destinées à traiter les pathologies cérébrales, et notamment pour l'utilisation de molécules susceptibles de traiter les désordres du SNC (Neuwelt et al., 2008, Lancet Neurol., 7, 84-96)*.*

L'une des raisons pouvant expliquer pourquoi aucun traitement efficace n'est actuellement disponible pour les principales pathologies cérébrales (cancer du cerveau, maladies de Parkinson et d'Alzheimer, accidents vasculaires cérébraux (AVC), etc.) est que les développeurs de candidats-médicaments destinés à traiter des pathologies du cerveau, réalisent en interne des programmes de recherche (*brain drug-discovery programs*) en investissant peu d'efforts dans la problématique du passage de la BHE et dans le ciblage préférentiel du SNC, et notamment du cerveau (*brain drug-targeting programs),* (Pardridge, 2003, Mol. Interv., 3, 90-105*).* Un candidat-médicament doit répondre à certaines règles structurales, physico-chimiques, pharmacochimiques et pharmacologiques afin d'avoir toutes les chances de devenir un médicament pour traiter une pathologie ou un trouble du SNC (Pajouhesh et al., 2005, NeuroRx, 2 (4), 541-553*).* Ainsi, dans le développement d'un candidat-médicament, la sélectivité et la spécificité (profilage pharmacologique) d'une molécule pour sa cible sont essentielles à son activité thérapeutique (efficacité). La biodisponibilité et la toxicité potentielle (profilage pharmaceutique) d'une molécule sont quant à elles cruciales pour son devenir en tant que médicament. Autrement dit, toute molécule susceptible de devenir un médicament destiné à traiter une pathologie ou un trouble du SNC doit d'une part passer au travers de la BHE, d'autre part, conserver son activité biologique, et présenter de bonnes propriétés de pharmacocinétique (PK), d'absorption, de distribution, de métabolisme et d'excrétion/élimination (ADME) et de pharmacodynamie (PD), avec une faible toxicité (Tox). De fait, la balance hydrophile/lipophile de la molécule en développement est particulièrement difficile à trouver pour les chimistes médicinaux dans cette aire thérapeutique du SNC.

Le problème majeur dans le traitement des troubles et pathologies du SNC réside donc dans le fait que les molécules administrées ne passent pas la BHE et ne peuvent donc pas atteindre leur(s) cible(s) dans le SNC. Les cellules endothéliales des vaisseaux et capillaires du SNC constitutives de la BHE font obstacle aux molécules qui ne peuvent pas passer du sang au tissu nerveux. En effet comme évoqué précédemment, ces cellules endothéliales et les pieds d'astrocytes qui les entourent constituent une barrière physique liée notamment à l'existence de jonctions serrées entre les cellules endothéliales qui limitent/empêchent tout passage/transport par la voie para-cellulaire, et également une barrière physiologique, puisque ces cellules disposent de systèmes d'efflux efficaces qui restreignent tout passage/transport par la voie trans-cellulaire. Ces propriétés limitent donc fortement le passage des substances du plasma sanguin vers l'espace extracellulaire cérébral.

En effet, certaines molécules, qui sont capables de franchir la BHE, sont expulsées activement du cerveau vers le système sanguin par des protéines de transport *multidrug resistant* (MDR). Ces systèmes d'efflux par transport actif (*active efflux transport,* AET) contrôlent généralement l'efflux actif des petites molécules depuis le cerveau vers le système sanguin. Le système d'AET modèle au niveau de la BHE est le transporteur *ATP Binding Cassette* (ABC) à savoir la glycoprotéine P (P-glycoprotein, P-gp) ; toutefois d'autres systèmes d'AET sont présents au niveau de la BHE tel que la *MDR-associated protein 1* (MRP1). La P-gp, qui est principalement localisée à la surface luminale des cellules endothéliales des capillaires cérébraux, est un élément essentiel dans la fonction de barrière physiologique de la BHE prévenant l'entrée dans le cerveau de la plupart des xénobiotiques mais également des candidats-médicaments et autres molécules d'intérêt thérapeutique pouvant être actives dans le SNC.

Une des priorités de la recherche, dans la découverte de molécules destinées à traiter, diagnostiquer ou imager les troubles ou les pathologies cérébrales, est donc de trouver des moyens permettant d'augmenter l'efficacité de passage des substances actives à travers la BHE.

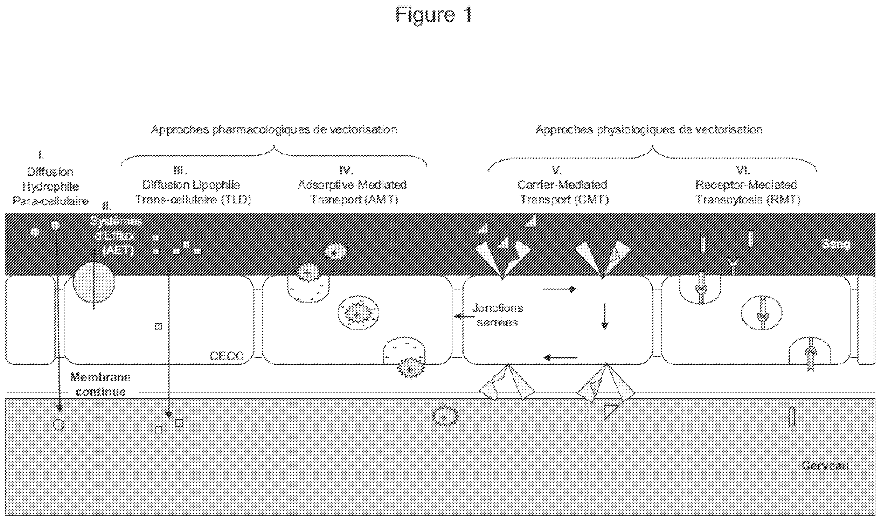

A cet égard, les stratégies de vectorisation de molécules au travers de la BHE, actuellement étudiées et utilisées par les développeurs de candidats-médicaments afin de permettre à une molécule d'intérêt thérapeutique d'atteindre le SNC, peuvent être divisées selon deux principales stratégies : les approches pharmacologiques et les approches physiologiques (Figure 1), (Pardridge, 2007, Pharm. Res., 24 (9), 1733-1744 *;* De Boer et al., 2007, Clin. Pharmacokinet., 46 (7), 553-576 *;* De Boer et al., 2007, Annu. Rev. Pharmacol. Toxicol., 47, 327-355 *;* Jones et al., 2007, Pharm. Res., 24 (9), 1759-1771)*.*

### Approches invasives

Les approches invasives peuvent être mise en oeuvre par injection intra-ventriculaire cérébrale directe de la substance active, injection intracérébrale ou infusion intrathécale, ou par perturbation de la BHE (rupture temporaire de l'intégrité de la BHE).
Le problème principal des approches neurochirurgicales par injection intra-ventriculaire, en dehors des coûts relatifs à l'acte neurochirurgical, est que le médicament n'est pas délivré directement au niveau du parenchyme cérébral mais dans le liquide céphalo-rachidien. L'infusion intra-ventriculaire implique le placement d'un cathéter dans les ventricules (Aird, 1984, Exp. Neurol., 86, 342-358). Cette technique très invasive n'est pas efficace pour le transport de substances actives dans le parenchyme cérébral. En effet, le volume d'écoulement depuis le liquide céphalo-rachidien jusqu'au parenchyme cérébral lors de la délivrance d'un médicament par infusion intra-ventriculaire est régi par une diffusion anormalement lente de sa convection (de son transport), car le cerveau ne possède pas de débit volumique intra-parenchymateux.
De même pour l'injection intracérébrale, la diffusion d'une substance active dans le cerveau décroît très rapidement depuis le site d'injection jusqu'au site de lésion. En effet, la concentration cérébrale d'une substance active diminue de 90% à une distance de 500 µm de son site d'injection.
L'infusion intrathécale implique le placement d'un cathéter dans le cerveau. Ce cathéter est connecté à une pompe qui délivre la substance active à un débit prédéfini. Du fait que le cerveau soit le seul organe qui n'ait pas de système lymphatique, servant normalement à ramener les fluides extracellulaires jusqu'à la circulation générale, la distribution d'une substance active par infusion intrathécale au niveau cérébral est très lente. Ceci diminue la concentration de la substance active au site de lésion.
De plus, les risques infectieux sont importants au cours de tels actes neurochirurgicaux, notamment de par la présence du cathéter. Dans ces conditions, le confort des patients n'est pas optimal.

La rupture temporaire de l'imperméabilité de la BHE est associée à une ouverture transitoire des jonctions serrées des cellules endothéliales des capillaires cérébraux.

C'est le cas des substances vasoactives comme les leukotriènes ou bradykinines (Baba et al., 1991, J. Cereb. Blood Flow Metab., 11, 638-643*).* Cette stratégie est également invasive et nécessite un accès artériel au niveau de la carotide chez des sujets/patients mis sous sédatifs. Le problème majeur rencontré par la rupture temporaire de l'intégrité de la BHE, outre les dépenses relatives à l'acte du radiologue pour l'accès à la carotide, est que la BHE ne reste ouverte que pendant une courte période de temps, limitant de fait la possibilité de délivrer en chronique un médicament. En outre, la rupture temporaire de la BHE permet aux protéines plasmatiques d'entrer dans le cerveau (alors que ces protéines peuvent être toxiques pour le cerveau) et peut également faciliter l'entrée d'agents infectieux. Ce type de rupture de la BHE peut donc conduire à des perturbations neuropathologiques chroniques et est associé à des risques importants d'infection (Salahuddin et al., 1988, Acta Neuropathol., 76, 1-10*).*

### Approches pharmacologiques de vectorisation

Les stratégies pharmacologiques pour le transport de molécules comprennent la diffusion trans-cellulaire de molécules rendues plus hydrophobes par l'addition de groupements lipidiques ou lipophiles sur la substance active (*Tiranscellular Lipophilic Diffusion* ou TLD) voire l'utilisation des liposomes (Zhou et al., 1992, J. Control. Release, 19, 459-486)*,* et le transport par adsorption ionique via des molécules vecteurs chargées positivement ou par cationisation de la molécule active (*Adsorptive-Mediated Transport* ou AMT).
L'addition d'un groupement lipidique ou lipophile permet la conversion chimique des molécules hydrophiles en molécules plus hydrophobes notamment au travers d'approches prodrogues. Cependant, la synthèse de tels composés conduit à des molécules qui dépassent le seuil de transport optimal pour traverser la BHE, notamment en ce qui concerne le poids moléculaire qui devient supérieur à la limite optimale de 450 Daltons (Pajouhesh et al., 2005, NeuroRx, 2 (4), 541-553)*.* Pour cette même raison, les liposomes ou même les petites vésicules (micelles, etc.) ou nanoparticules (nanosphères, nanocapsules) sont généralement de trop grande taille, pas assez spécifiques pour la BHE, et par conséquent relativement peu efficaces pour le transport de molécules d'intérêt thérapeutique (ou d'agents d'imagerie ou de diagnostic, ou de toute autre molécule comme une sonde moléculaire) à travers la BHE (Levin, 1980, J. Med. Chem., 23, 682-684 *;* Schackert et al., 1989, Selective Cancer Ther., 5, 73-79*).* De plus, ce type de système vésiculaire présente généralement des effets toxiques non négligeagles au niveau cérébral. Ainsi, les principaux problèmes rencontrés par les technologies de lipidisation sont leur faible spécificité pour cibler et traverser spécifiquement la BHE par rapport à d'autres membranes cellulaires, la diminution des valeurs plasmatiques de l'aire sous la courbe de la drogue (ASC), et leur utilisation généralement limitée à la vectorisation de petites molécules.
Dans les approches d'AMT (addition d'un groupement cationique via couplage covalent ou cationisation directe de la drogue), le principal problème rencontré est la faible spécificité pour cibler et traverser spécifiquement la BHE par rapport à d'autres membranes cellulaires. En effet, l'AMT est basée sur des molécules cationiques s'adsorbant sur des cellules dont la membrane est chargée négativement, ce qui est le cas de la plupart des cellules. La diminution des valeurs plasmatiques de l'ASC de la drogue, leur utilisation généralement limitée à la vectorisation de petites molécules, et leur cytotoxicité sont autant de facteurs qui pénalisent l'approche de vectorisation via AMT.

### Approches physiologiques de vectorisation

Les stratégies fondées sur des approches physiologiques de vectorisation consistent à exploiter les différents mécanismes de transport naturel au niveau de la BHE. Ces mécanismes de transport actif de molécules au travers de la BHE se font soit via couplage à un substrat spécifique d'un récepteur ou par mimétisme moléculaire avec le substrat spécifique d'un récepteur (*Carrier-Mediated Transport* ou CMT), soit via couplage ou fusion à un ligand ciblant spécifiquement un récepteur (*Receptor-Mediated Transport* ou RMT).

A titre d'exemple des molécules comme la L-DOPA (maladie de Parkinson), le melphalan (cancer du cerveau), l'α-méthyl-DOPA (hypertension artérielle) et la gabapentine (épilepsie) passent dans le cerveau par CMT via les *large neutral amino-acid transporters 1 et 2* (LAT1 et LAT2), (Pardridge, 2003, Mol. Interv., 3, 90-105*).* Ces molécules ont des structures chimiques proches de la phénylalanine, l'un des substrats naturels de LAT1/2. Toutefois, les principaux problèmes rencontrés par les approches CMT sont leur grande sélectivité/spécificité pour des conjugués qui imitent/miment étroitement le substrat du récepteur/transporteur endogène, et par conséquent leur utilisation qui reste limitée à la vectorisation de petites molécules.
Le RMT fait appel à un système de transport récepteur-dépendant. La vectorisation est réalisée via des mécanismes d'endocytose par le ciblage des récepteurs/transporteurs endogènes présents au niveau des capillaires cérébraux. Parmi les différents récepteurs humains de la BHE impliqués dans le RMT, on recense notamment: le récepteur à la transferrine (TfR), le récepteur à l'insuline (IR), les récepteurs aux *low-density lipoproteins* (LDL) permettant le transport du cholestérol dont le récepteur au LDL (LDLR) et les membres de la famille des *low-density lipoprotein receptor-related protein* (LRP), ou encore le récepteur à l*'insulin-like growth factor* (IGFR), le récepteur à la toxine diphtérique (DTR) ou *heparin binding epidermal growth factor-like growth factor* (HB-EGF), ainsi que les *scavenger receptor* (SCAV-Rs) dont le *scavenger receptor class B type I* (SR-BI). Dans le RMT, les récepteurs sur la membrane d'une cellule endothéliale de la BHE lient leur ligand, ce qui entraîne l'endocytose du complexe constitué du récepteur/transporteur et de son ligand dans une vésicule qui se forme à la surface de la cellule et pénètre ensuite dans la cellule endothéliale de la BHE. Le complexe ligand/récepteur peut passer au travers de la cellule endothéliale (transcytose), et par conséquent traverser ainsi la BHE pour agir dans le tissu nerveux. Ce processus de RMT ne dépend pas de la taille de ce qui est endocyté. Ainsi, le RMT est un mécanisme qui permet le transport depuis le sang jusque dans le cerveau de molécules telles que l'insuline, des protéines transporteuses de fer, le cholestérol, divers dérivés peptidiques et protéines, etc. Par exemple, la transferrine est utilisée comme vecteur, ligand du TfR présent sur la BHE (Jefferies et al., 1984, Nature, 312, 162-163 *;* Friden et al., 1983, Science, 259, 373-377 *;* Friden, 1994, Neurosurgery, 35, 294-298*),* et la molécule à transporter (substance active) est couplée à la transferrine (vecteur ligand). Bien que cette stratégie de vectorisation à l'aide d'une macromolécule permette une augmentation du passage des molécules d'intérêt conjuguées à travers la BHE, elle présente quelques inconvénients. D'abord, le couplage de la molécule au vecteur se fait généralement par des méthodes d'expression génétique (fusion) limitant ainsi le nombre de molécules à transporter à seulement des polypeptides ou des protéines. Ensuite, le système de couplage de la molécule au vecteur est assez complexe, un couplage chimique ou biochimique traditionnel ne permet pas d'obtenir des systèmes macromoléculaires bien définis d'un point de vue structurel et moléculaire. De plus, la compétition potentielle entre les conjugués et les ligands endogènes pour le récepteur ciblé peut conduire soit à une inhibition du processus physiologique de RMT, soit à une diminution de la concentration de ligands endogènes nécessaire pour le bon fonctionnement du cerveau. Les récepteurs RMT sont impliqués dans des processus de signalisation cellulaire au niveau cérébral et les conjugués pourraient potentiellement interférer avec ces processus.

La demande de brevet internationale n° WO2010/046588 décrit pour la première fois des peptides ou pseudo-peptides qui lient le LDLR humain et sont capables de véhiculer à travers la BHE des substances pouvant être de masse et/ou de volume important(s).

La présente demande concerne de nouveaux peptides liant le LDLR humain, optimisés pour le transport de molécules.

### RESUME DE L'INVENTION

La présente invention fournit des peptides ou pseudo-peptides optimisés capables de véhiculer à travers les membranes cellulaires, et plus spécifiquement la BHE, des substances pouvant être de masse et/ou de volume important(s). L'invention permet ainsi d'améliorer la biodistribution voire la biodisponibilité de molécules d'intérêt, et notamment d'améliorer leur accès (ciblage) au SNC.

Dans la demande WO2010/046588, les inventeurs ont mis au point des dérivés peptidiques capables de lier le LDLR humain. Les inventeurs ont montré que ces dérivés étaient capables de traverser la BHE. Les inventeurs ont également montré que ces dérivés permettaient de véhiculer, dans les cellules de la BHE, des molécules d'intérêt thérapeutique ou diagnostique.

Poursuivant leurs recherches, les inventeurs sont parvenus à concevoir et tester de nouveaux peptides présentant des propriétés avantageuses pour le transport de molécules. Ces nouveaux peptides, capables de lier le LDLR sans compétition avec le ligand naturel, et donc sans interférence avec le transport du LDL endogène, représentent de nouveaux produits (vecteurs) particulièrement avantageux pour la conception et la vectorisation de médicaments ou d'agents de diagnostic ou d'imagerie, notamment pour atteindre le SNC.

Un objet de l'invention réside ainsi dans un peptide ou pseudo-peptide, caractérisé en ce qu'il répond à la formule générale (I) suivante :

A1-Met-A2-Arg-Leu-Arg-A3-Cys (I)

dans laquelle A1 désigne la cystéine ou un de ses analogues ou isostères, A2 désigne la proline ou un de ses analogues ou isostères, et A3 désigne la glycine ou un des ses analogues ou isostères. Etant entendu que le résidu A2 est un analogue de proline lorsque le résidu A1 est une cystéine, et qu'il est capable de lier un récepteur humain aux lipoprotéines de faible densité (hLDLR). Selon des modes préférés, A1 désigne la cystéine (Cys) ou un de ses analogues choisi parmi la (D)-cystéine, la Pénicillamine (Pen) et la (D)-pénicillamine ((D)-pen) ; A2 désigne la proline (Pro) ou un de ses analogues choisi parmi l'acide pipécolique (Pip) et l'acide thiazolidine-4-carboxylique (Thz) ; et/ou A3 désigne la glycine (Gly), ou la sarcosine (Sar).
Un objet préféré de l'invention réside dans un peptide ou pseudo-peptide de formule générale (I') suivante :

A1 -Met-A2-Arg-Leu-Arg-Gly-Cys (I')

dans laquelle A1 désigne la cystéine ou un de ses analogues choisi de préférence parmi (D)-cys, Pen et (D)-pen ; et A2 désigne la proline ou un de ses analogues choisi de préférence parmi Pip et Thz.
Un autre objet particulier de l'invention réside dans un peptide ou pseudo-peptide de formule générale (I") suivante :

A1-Met-A2-Arg-Leu-Arg-Ala-Cys (I')

dans laquelle A1 désigne la cystéine ou un de ses analogues choisi de préférence parmi (D)-cys, Pen et (D)-pen ; et A2 désigne la proline ou un de ses analogues choisi de préférence parmi Pip et Thz. L'alanine (Ala) peut être de configuration L ou D.

Les peptides de l'invention présentent avantageusement la capacité de lier le récepteur humain aux LDL (hLDLR), avec une affinité élevée. Ils possèdent par ailleurs une taille réduite, typiquement inférieure à 10 acides aminés, ce qui est particulièrement avantageux. Des exemples particuliers de tels peptides sont notamment les peptides de séquences SEQ ID NOs : 1 à 10.

Un autre objet de l'invention concerne l'utilisation d'un peptide ou pseudo-peptide tel que défini ci-avant pour la préparation d'une composition pharmaceutique ou diagnostique pour vectoriser une substance active ou d'intérêt en diagnostic, imagerie ou thérapie.

Un autre objet de l'invention concerne l'utilisation d'un peptide ou pseudo-peptide tel que défini ci-avant pour augmenter l'activité biologique ou diminuer la toxicité d'une substance active ou d'intérêt à laquelle il est couplé.

Un autre objet de l'invention concerne un peptide ou pseudo-peptide tel que défini ci-avant pour son utilisation pour vectoriser une substance active ou d'intérêt en diagnostic, imagerie ou thérapie.

Un autre objet de l'invention concerne un peptide ou pseudo-peptide tel que défini ci-avant pour son utilisation pour augmenter l'activité biologique ou diminuer la toxicité d'une substance active ou d'intérêt à laquelle il est couplé.

L'invention a encore pour objet tout composé conjugué de formule (II) suivante :

VxDy (II)

dans laquelle V représente un peptide ou pseudo-peptide tel que défini ci-avant, D représente une substance active ou d'intérêt, et x et y sont des nombres entiers compris entre 1 et 5.

L'invention concerne également tout composé conjugué de formule (III) suivante :

VxLzDy (III)

dans laquelle V représente un peptide ou pseudo-peptide tel que défini ci-avant, L représente un bras espaceur, D représente une substance active ou d'intérêt, x et y sont des nombres entiers compris entre 1 et 5 et z est un entier compris entre 1 et 10.

Un autre objet de l'invention concerne une composition pharmaceutique comprenant au moins un composé conjugué tel que défini ci-avant et un ou des excipients pharmaceutiquement acceptables.

Un autre objet de l'invention concerne une composition diagnostique caractérisée en ce qu'elle comprend un agent de diagnostic ou d'imagerie médicale constitué d'un composé conjugué tel que défini ci-avant.

Un autre objet de l'invention concerne une méthode pour améliorer ou permettre le passage d'une molécule à travers la BHE, comprenant le couplage de cette molécule à un peptide ou pseudo-peptide tel que défini ci-dessus.

Un autre objet de l'invention réside dans une méthode améliorée de traitement d'une pathologie chez un sujet par un médicament, l'amélioration consistant à coupler ce médicament à un peptide ou pseudo-peptide tel que défini ci-dessus.

L'invention est utilisable chez tout mammifère, notamment tout être humain.

### LEGENDE DES FIGURES

Figure 1 Schéma illustrant les différents modes de passage de molécules naturelles ou pharmacologiques à travers la BHE, adapté d'après Abbott et Romero, 1996, Mol. Med. Today, 2 (3), 106-113*.*
Figure 2 Schéma comparatif de la synthèse en tandem et de la synthèse via un *linker* d'un conjugué vecteur/molécule d'intérêt thérapeutique.
Figure 3
   A - Schéma du plasmide utilisé pour cloner les hLDLR et mLDLR.
   B - Schéma représentant la protéine de fusion exprimée par les cellules transfectées.

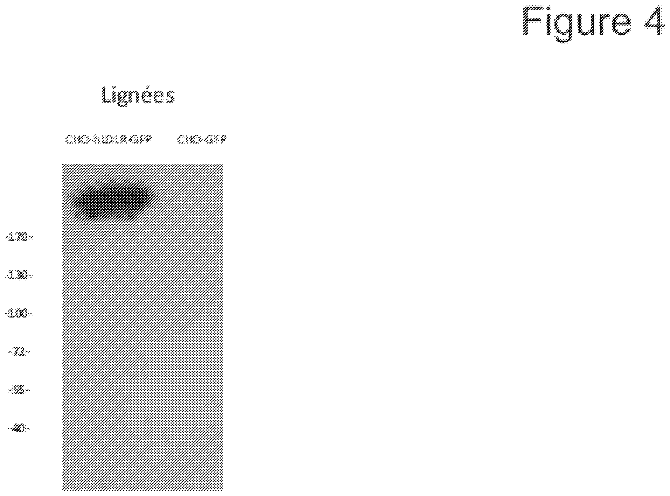

Figure 4 Western blot réalisé sur des lignées cellulaires CHO exprimant de manière constitutive les protéines de fusion hLDLR-GFP ou GFP (utilisée comme contrôle). Une bande de 190 kDa correspondant à la taille de la protéine de fusion hLDLR-GFP est détectée avec l'anticorps anti-hLDLR.

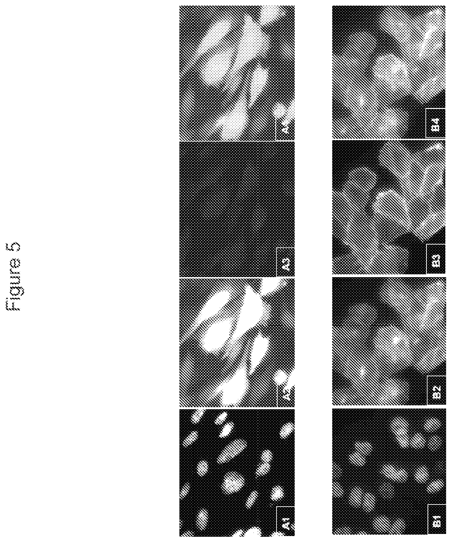

Figure 5 Immunocytochimie sur cellules CHO non perméabilisées, exprimant de manière stable soit A - la GFP seule (contrôle), soit B - la construction hLDLR-GFP. Les noyaux cellulaires sont marqués au Hoechst (bleu, A1 et B1). La fluorescence de la GFP est visible en vert (A2 et B2), celle du marquage du domaine extracellulaire du hLDLR par l'anticorps anti-hLDLR est visible dans le rouge (A3 et B3) et la superposition des marquages rouges et verts est visible en jaune/orange (A4 et B4). Noter que seules les cellules transfectées de manière stable avec la construction hLDLR-GFP expriment le récepteur à la membrane (B3).
Figure 6
   A - Cellules de la lignée CHO-hLDLR-GFP exprimant le hLDLR-GFP (vert) incubées avec du DiI-LDL (rouge), la superposition des marquages rouges et vert est visible en jaune/orange, noter le fort marquage des cellules.
   B - Cellules d'une lignée CHO-TfR-GFP exprimant le hTfR-GFP (vert) incubées avec du DiI-LDL (rouge) : absence de liaison et d'endocytose du DiI-LDL.
   C - Cellules de la lignée CHO-hLDLR-GFP exprimant le hLDLR-GFP (vert) incubées avec de la transferrine couplée à du *Texas Red* (rouge) : absence de liaison et d'endocytose du ligand Tf.
   Noter la différence d'intensité de marquage entre A d'une part, B et C d'autre part, où seul le marquage des récepteurs hTfr et hLDLR fusionnés à la GFP est détecté.

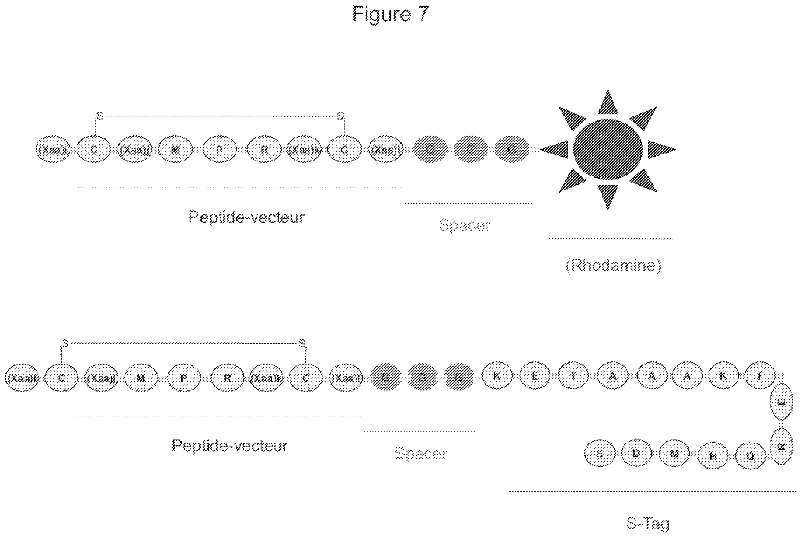

Figure 7 Schéma général des peptides synthétisés conjugués à la rhodamine ou au S-Tag, avec bras espaceur en C-terminal (C-term).

### DESCRIPTION DETAILLEE DE L'INVENTION

L'invention concerne des dérivés peptidiques capables de lier le LDLR humain et leur utilisation dans le domaine pharmaceutique, notamment pour transporter, dans les cellules de la BHE, des molécules d'intérêt thérapeutique ou diagnostique.

Le LDLR humain est une protéine transmembranaire de 839 acides aminés comprenant trois régions : la région extracellulaire (1-768), la région transmembranaire (768-790) et la région cytoplasmique (790-839). La région extracellulaire est divisée en deux sous-régions : celle de liaison des LDL (1-322) et celle en dehors de la zone de liaison des LDL (322-768) (voir WO2007/014992*).*

Le cerveau a un besoin important de LDL pour son bon fonctionnement. Les ligands naturels au LDLR sont le LDL et plus particulièrement l'apolipoprotéine B (ApoB) et l'apolipoprotéine E (ApoE) constitutives des particules de LDL permettant ainsi le transport de cholestérol, contenu dans ces particules, au travers des membranes cellulaires et plus particulièrement de la BHE.

Il a ainsi été démontré que le LDLR permettait la transcytose de particules de LDL au travers de la BHE (Dehouck et al., 1997, J. Cell Biol., 138 (4), 877-889), via un processus de RMT, dans des vésicules endosomiques particulières qui empêchent la fusion au lysosome. Ces lipoprotéines, ayant passé la BHE par transcytose, sont alors captées par les neurones et/ou les astrocytes (Spencer et al., 2007, Proc. Natl. Acad. Sci. USA, 104 (18), 7594-7599*).* Cette propriété a été utilisée pour vectoriser des molécules d'intérêt thérapeutique par des nanoparticules sur lesquelles sont conjuguées des apolipoprotéines entières (constitutives du LDL) (Kreuter et al., 2007, J. Control. Release, 118, 54-58*).* Dans cette étude, toutefois, une apolipoprotéine entière a été utilisée, et sous forme couplée à une nanoparticule afin de mimer la structure de la particule LDL.

La demande de brevet internationale n° WO2010/046588 décrit pour la première fois des peptides ou pseudo-peptides qui lient le LDLR humain et sont capables de véhiculer à travers la BHE des substances pouvant être de masse et/ou volume importants.

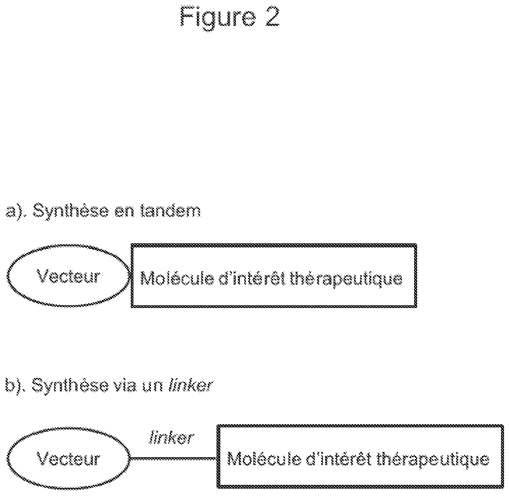

La présente demande concerne de nouveaux peptides liant le LDLR humain, optimisés pour le transport de molécules. Les peptides ou pseudo-peptides vecteurs peuvent être aisément synthétisés par voie chimique, et la plupart des molécules d'intérêt thérapeutique, ou des agents d'imagerie ou de diagnostic peuvent être couplées au peptide ou pseudo-peptide vecteur de façon simple et efficace au travers d'une stratégie prodrogue via un bras espaceur (synthèse via *linker)* ou par couplage direct (synthèse en tandem) entre les deux entités (Figure 2). Les peptides et pseudo-peptides de l'invention sont conçus pour adopter une configuration cyclique, et donc plus résistante à la protéolyse. Par ailleurs, les peptides et pseudo-peptides de l'invention se lient au LDLR sans compétition avec le ligand naturel.

Les peptides ou pseudo-peptides de l'invention peuvent être utilisés comme vecteurs de molécules d'intérêt thérapeutique, ou d'agents d'imagerie ou de diagnostic, ou de toute autre molécule comme une sonde moléculaire, dans le traitement, l'imagerie et/ou le diagnostic de pathologies neurologiques, ainsi que de pathologies infectieuses ou cancéreuses cérébrales ou non.

Les peptides ou pseudo-peptides décrits dans la présente invention possèdent la capacité de cibler des récepteurs/transporteurs cellulaires, des types cellulaires particuliers et/ou de passer les membranes cellulaires notamment celles des barrières physiologiques du cerveau et plus particulièrement la BHE ou la barrière sang-rétine (BSR).

Les peptides ou pseudo-peptides décrits dans la présente invention possèdent la capacité de cibler des récepteurs/transporteurs cellulaires, des types cellulaires particuliers, notamment des cellules cancéreuses, du tissu nerveux ou non et/ou de passer les membranes cellulaires notamment celles des barrières physiologiques du SNC et plus particulièrement la barrière hémato-tumorale (BHT) au niveau des tumeurs du tissu nerveux.

Les peptides ou pseudo-peptides décrits dans la présente invention possèdent la capacité de cibler des récepteurs/transporteurs cellulaires, des types cellulaires particuliers et/ou de passer les membranes cellulaires, notamment celles des barrières physiologiques du SNC, pour traiter plus particulièrement des pathologies infectieuses cérébrales ou autres, de type bactériennes, virales, parasitaires ou fongiques.

Les peptides ou pseudo-peptides décrits dans la présente invention possèdent la capacité de se fixer sur un LDLR murin ou humain de la membrane cellulaire et de traverser ladite membrane grâce à ce récepteur par transcytose.

Les peptides ou pseudo-peptides décrits dans la présente invention possèdent la capacité de se fixer sur le LDLR à la surface des membranes cellulaires des barrières physiologiques du cerveau de type murin et humain et de traverser ladite barrière physiologique grâce au LDLR par RMT.

Un objet de l'invention concerne ainsi des peptide ou pseudo-peptides, caractérisés en ce qu'ils répondent à la formule générale (I) suivante :

A1-Met-A2-Arg-Leu-Arg-A3-Cys (I)

dans laquelle :
- A1 désigne la cystéine ou un de ses analogues ou isostères,
- A2 désigne la proline ou un de ses analogues ou isostères, et
- A3 désigne la glycine ou un des ses analogues ou isostères.

Plus particulièrement, le group A1 désigne un résidu choisi parmi la cystéine (Cys, C), de configuration D ou L, ou un dérivé de celle-ci choisi parmi l'acide 2-amino-3-mercaptopropanoïque et ses dérivés S-substitués, la S-acétylcystéine ou acide 2-amino-3-(acétylthio)propanoïque, la sélénocystéine (Sec, U) ou acide 2-amino-3-(séléno)propanoïque, le cystéinol, l'acide 3-mercaptopropanoïque (Mpa), ou la pénicillamine (Pen), de configuration L ou D.
Dans un mode préféré, le groupe A1 est choisi parmi la cystéine ou la pénicillamine, de configuration L ou D.

Le group A2 désigne plus préférentiellement un résidu choisi parmi la proline (Pro, P) ou acide pyrolidine-2-carboxylique, l'homoproline ou acide 2-(2-pyrrolidinyl)éthanoïque, la 3-hydroxyproline (3Hyp), la 4-hydroxyproline (4Hyp), la 3-méthylproline, la 3,4-déshydroproline, la 3,4-méthanoproline, la 4-aminoproline, la 4-oxoproline, la thioproline ou acide thiazolidine-4-carboxylique (Thz), l'acide 2-oxothiazolidine-4-carboxylique, l'acide indoline-2-carboxylique (Idc), l'acide pipécolique (Pip) ou acide piperidine-2-carboxylique, l'acide nipécotique (Nip) ou acide piperidine-3-carboxylique, l'acide 4-oxopipécolique, l'acide 4-hydroxypipécolique, l'acide amino-1-cyclohexane carboxylique, le prolinol.
Dans un mode préféré, le groupe A2 est choisi parmi la proline, l'acide pipécolique (Pip) ou l'acide thiazolidine-4-carboxylique (Thz).

Le group A3 désigne plus préférentiellement un résidu choisi parmi la glycine (Gly, G) ou acide 2-aminoéthanoïque, la sarcosine (Sar) ou N-méthylglycine (MeGly), la N-éthylglycine (EtGly), l'allylglycine (allylGly) ou acide 2-aminopent-4-énoïque, la 2-cyclopentylglycine (Cpg), la 2-cyclohexylglycine (Chg), la 2,2-dipropylglycine (Dpg), la 2-(3-indolyl)glycine (IndGly), la 2-indanylglycine (Igl), la 2-néopentylglycine (NptGly), la 2-octylglycine (OctGly), la 2-propargylglycine (Pra) ou acide 2-amino pent-4-ynoïque, la 2-phénylglycine (Phg), la 2-(4-chlorophényl)glycine, l'azaglycine (AzGly), ou le glycinol ou 2-aminoéthanol.
Dans un mode préféré, le groupe A3 est choisi parmi la glycine et la sarcosine.

Parmi les peptides ou pseudo-peptides de formule (I), on préfère tout particulièrement les peptides dans lesquels le group A3 est la glycine. Ainsi, un objet particulier de l'invention concerne des peptides de formule (I') suivante :

A1-Met-A2-Arg-Leu-Arg-Gly-Cys (I')

dans laquelle A1 désigne la cystéine ou un de ses analogues ou isostères, de préférence A1 désigne Cys, (D)-cys, Pen, ou (D)-pen ; et A2 désigne un analogue de la proline, de préférence A2 désigne Pip ou Thz.

Des exemples particuliers de peptides selon l'invention sont décrits ci-dessous :
SEQ ID NO : 1, (D)-cys-Met-Pip-Arg-Leu-Arg-Gly-Cys ;
SEQ ID NO : 2, (D)-pen-Met-Pip-Arg-Leu-Arg-Gly-Cys ;
SEQ ID NO : 3, Pen-Met-Pip-Arg-Leu-Arg-Gly-Cys ;
SEQ ID NO : 4, (D)-cys-Met-Thz-Arg-Leu-Arg-Gly-Cys ;
SEQ ID NO : 5, (D)-pen-Met-Thz-Arg-Leu-Arg-Gly-Cys ;
SEQ ID NO : 6, Pen-Met-Thz-Arg-Leu-Arg-Gly-Cys ;
SEQ ID NO : 7, (D)-cys-Met-Thz-Arg-Leu-Arg-Sar-Cys ;
SEQ ID NO : 8, (D)-pen-Met-Thz-Arg-Leu-Arg-Sar-Cys ;
SEQ ID NO : 9, Pen-Met-Thz-Arg-Leu-Arg-Sar-Cys ;
SEQ ID NO : 10, (D)-cys-Met-Pro-Arg-Leu-Arg-(D)-ala-Cys.

Les résultats obtenus par le demandeur montrent que les peptides de l'invention possèdent une affinité améliorée pour le LDLR. Ainsi, par rapport au peptide de référence SEQ ID NO : 17, les peptides testés de formule (I) présentent tous une affinité améliorée, comme cela ressort du Tableau 1 (EXEMPLE III). Ces résultats sont d'autant plus remarquables que la modification d'autres résidus dans la séquence, comme par exemple le remplacement des résidus arginine, conduit à une chute substantielle d'affinité (voir peptides de référence SEQ ID NO : 11-16).
SEQ ID NO : 11, (D)-Cys-Met-Pro-hArg-Leu-Arg-Gly-Cys ;
SEQ ID NO : 12, (D)-Cys-Met-Pro-Agb-Leu-Arg-Gly-Cys ;
SEQ ID NO : 13, (D)-Cys-Met-Pro-Agp-Leu-Arg-Gly-Cys ;
SEQ ID NO : 14, (D)-Cys-Met-Pro-Cit-Leu-Arg-Gly-Cys ;
SEQ ID NO : 15, (D)-Cys-Met-Pro-Arg-Leu-Cit-Gly-Cys ;
SEQ ID NO : 16, (D)-Cys-Met-Pro-Arg-Leu-Arg(NO₂)-Gly-Cys ;
SEQ ID NO : 17, (D)-Cys-Met-Pro-Arg-Leu-Arg-Gly-Cys.

Ces résultats sont également particulièrement remarquables compte tenu de la taille réduite des peptides (les peptides ci-dessus comportent 8 acides aminés), qui constitue un atout supplémentaire dans leur utilisation industrielle.

Comme indiqué précédemment, les peptides ou pseudo-peptides cycliques de l'invention peuvent comprendre des liaisons peptidiques, non-peptidiques et/ou peptidiques modifiées. Dans un mode préféré, les peptides ou pseudo-peptides comprennent au moins une liaison peptidomimétique, choisie de préférence parmi l'intercalation d'un groupe méthylène (-CH₂-) ou phosphate (-PO₂-), aminé secondaire (-NH-) ou oxygène (-O-), des alpha-azapeptides, alpha-alkylpeptides, N-alkylpeptides, phosphonamidates, depsipeptides, hydroxyméthylènes, hydroxyéthylènes, dihydroxyéthylènes, hydroxyéthylamines, retro-inverso, méthylèneoxy, cétométhylène, esters, phosphinates, phosphiniques, phosphonamides, analogues carba.

Par ailleurs, dans un mode particulier de mise en oeuvre, les peptides ou pseudo-peptides de l'invention comprennent une fonction N-term et/ou C-term respectivement protégée, par exemple, par une acylation, ou par une amidation ou une estérification.

Les peptides ou pseudo-peptides de l'invention peuvent être synthétisés par toute technique connue en soi de l'homme du métier (synthèse chimique, biologique, génétique, etc.). Ils peuvent être conservés tels quels, ou formulés en présence d'une substance d'intérêt ou de tout excipient acceptable.

Pour les synthèses chimiques, sont utilisés des appareils commerciaux permettant d'incorporer aussi bien des acides aminés naturels que non naturels, tels que les énantiomères D et des résidus ayant des chaînes latérales avec des hydrophobicités et des encombrements stériques différents de ceux de leurs homologues naturels (acides aminés dits exotiques c'est-à-dire non codés), ou une séquence peptidique contenant une ou plusieurs liaisons peptidomimétiques pouvant inclure notamment l'intercalation d'un groupe méthylène (-CH₂-) ou phosphate (-PO₂-), d'une amine secondaire (-NH-) ou d'un oxygène (-O-), d'un N-alkylpeptide.

Au cours de la synthèse, il est possible d'introduire diverses modifications chimiques, comme par exemple, mettre en N-term, C-term ou sur une chaîne latérale un dérivé lipidique (ou phospholipidique) ou un constituant d'un liposome ou d'une nanoparticule, de façon à pouvoir incorporer le peptide ou pseudo-peptide de l'invention au sein d'une membrane lipidique telle que celle d'un liposome constitué d'une ou plusieurs couches ou bicouches lipidiques, ou d'une nanoparticule.

On peut également obtenir les peptides de l'invention, ou partie de ceux-ci de nature protéique à partir d'une séquence d'acide nucléique codant pour celui-ci. La présente invention a aussi pour objet une molécule d'acide nucléique comprenant, ou constituée par, une séquence nucléique codant pour un peptide tel que défini ci-avant. Plus particulièrement l'invention concerne une molécule d'acide nucléique comprenant au moins une séquence codant pour un peptide de formule générale (I). Ces séquences d'acides nucléiques peuvent être des ADN ou ARN et être associées à des séquences de contrôle et/ou être insérées dans des vecteurs d'expression biologiques.

Le vecteur d'expression biologique utilisé est choisi en fonction de l'hôte dans lequel il sera transféré. Il peut s'agir par exemple d'un plasmide, cosmide, virus, etc. Ces acides nucléiques et vecteurs d'expression biologiques constituent des objets particuliers de l'invention, et sont utiles pour produire les peptides de l'invention, ou partie de ceux-ci de nature protéique, dans un hôte cellulaire. La préparation de ces vecteurs d'expression biologiques ainsi que la production ou l'expression dans un hôte des peptides peuvent être réalisées par les techniques de biologie moléculaire et de génie génétique bien connues de l'homme de l'art.

Un autre objet de l'invention réside dans l'utilisation d'un peptide ou pseudo-peptide cyclique tel que défini précédemment, comme vecteur pour le transfert/transport de molécules d'intérêt thérapeutique, ou d'agents d'imagerie ou de diagnostic, ou de toute autre molécule.

Un autre objet de l'invention réside dans l'utilisation d'un peptide ou pseudo-peptide cyclique tel que défini précédemment pour la préparation d'un médicament capable de traverser la BHE.

Un autre objet de l'invention concerne une méthode pour permettre ou améliorer le passage d'une molécule à travers la BHE, comprenant le couplage de la molécule à un peptide ou pseudo-peptide de l'invention.

Ainsi, un objet particulier de l'invention concerne un composé conjugué de formule (II) suivante :

VxDy (II)

dans laquelle V représente un peptide ou pseudo-peptide de l'invention, D représente une substance active ou d'intérêt, et x et y sont des nombres entiers compris entre 1 et 5. Dans un mode particulier, x et y sont égal à 1, x est supérieur à y, ou y est supérieur à x.

Un autre objet particulier de l'invention concerne un composé conjugué de formule (III) suivante :

VxLzDy (III)

dans laquelle V représente un peptide ou pseudo-peptide de l'invention, L représente un bras espaceur, D représente une substance active ou d'intérêt, x et y sont des nombres entiers compris entre 1 et 5 et z est un entier compris entre 1 et 10. Dans un mode particulier, x=z=y=1 ou x=z>y ou y=z>x ou z>x>y.

La substance active ou d'intérêt peut être toute molécule d'intérêt pharmaceutique, notamment thérapeutique, un agent de diagnostic ou d'imagerie médicale, ou une sonde moléculaire. Il peut s'agir en particulier de toute entité chimique ayant un intérêt biologique telle qu'une petite molécule chimique (antibiotique, antiviral, immuno-modulateur, anticancéreux, anti-inflammatoire, etc.), un peptide ou polypeptide, une protéine (enzyme, hormone, cytokine, apolipoprotéine, facteur de croissance, antigène, anticorps ou une partie d'anticorps), un acide nucléique (acide ribonucléique ou acide désoxyribonucléique d'origine humaine, virale, animale, eucaryote ou procaryote, végétale, synthétique, etc., pouvant avoir une taille variable, allant du simple oligonucléotide au génome ou fragment de génome), un génome viral ou un plasmide, un ribozyme, un marqueur ou traceur. De manière générale, la « substance d'intérêt » peut être tout principe actif de médicament, qu'il s'agisse d'un produit chimique, biochimique, naturel ou synthétique. L'expression « petite molécule chimique » désigne une molécule d'intérêt pharmaceutique ayant un poids moléculaire de 1000 Daltons au maximum, typiquement compris entre 300 et 700 Daltons.

Un autre objet particulier de l'invention concerne un composé de formule (IV) suivante :

VxLz (IV)

dans laquelle V représente un peptide ou pseudo-peptide de l'invention, L représente un bras espaceur, x est un nombre entier compris entre 1 et 5 et z est un entier compris entre 1 et 10. Dans un mode particulier, x=z=1 ou z>x.

Dans les composés conjugués de l'invention, le couplage entre V et D, ou entre V et L d'une part et entre L et D d'autre part, peut être réalisé par tout moyen de liaison acceptable compte tenu de la nature chimique, de l'encombrement et du nombre de substance(s) active(s) et de peptide(s) ou pseudo-peptide(s) associés. Le couplage peut ainsi être réalisé par une ou plusieurs liaisons covalentes, ioniques, hydrogènes, hydrophobes ou de Van der Waals, clivables ou non en milieu physiologique ou à l'intérieur de cellules. Par ailleurs, D peut être couplé à V, le cas échéant via L, au niveau de différents groupes réactifs, et notamment d'une ou des extrémités N-term et/ou C-term de V et/ou au niveau d'un ou plusieurs groupements réactifs portés par les chaînes latérales des acides aminés naturels ou non, constitutifs de V.

Le couplage peut être effectué en n'importe quel site du peptide ou pseudo-peptide, dans lequel des groupements fonctionnels tels que -OH, -SH, -CO₂H, -NH₂, -SO₃H,-PO₂H sont naturellement présents ou ont été introduits. Ainsi, une molécule d'intérêt thérapeutique, ou un agent de diagnostic (ou d'imagerie médicale) ou toute autre molécule comme une sonde moléculaire peut être liée (couplée) au peptide ou pseudo-peptide vecteur de l'invention par une liaison covalente soit au niveau des extrémités N-term ou C-term, soit au niveau des groupements réactifs portés par les chaînes latérales des acides aminés naturels ou non, de cette séquence peptidique.

De même, le couplage peut être effectué en n'importe quel site de la substance active ou d'intérêt (molécule d'intérêt thérapeutique, agent de diagnostic ou d'imagerie médicale, toute autre molécule comme une sonde moléculaire), où par exemple des groupements fonctionnels tels que -OH, -SH, -CO₂H, -NH₂, -SO₃H, -PO₂H sont naturellement présents ou ont été introduits.

Il est préférable que l'interaction soit suffisamment solide pour que le peptide ne se dissocie pas de la substance active avant d'avoir atteint son site d'action. Pour cette raison, le couplage préféré selon l'invention est un couplage covalent, mais il pourrait cependant s'agir d'un couplage non covalent. La substance d'intérêt peut être couplée directement au peptide (synthèse en tandem) soit à l'une de ces extrémités terminales (N-term ou C-term), soit au niveau d'une chaîne latérale d'un des acides aminés constitutifs de la séquence (Majumdar and Siahaan, Med Res Rev.*, Epub ahead of point*)*.* La substance d'intérêt peut aussi être couplée indirectement par le biais d'un bras de liaison ou bras espaceur (synthèse via *linker*), soit à l'une des extrémités terminales des peptides, soit au niveau d'une chaîne latérale d'un des acides aminés constitutifs de la séquence (Figure 2). On peut citer comme moyen de couplage chimique covalent, faisant appel ou pas à un bras espaceur, ceux choisis parmi des agents bi- ou multifonctionnels contenant des groupements alkyle, aryle ou peptidique par des esters, aldéhydes ou acides d'alkyle ou d'aryle, des groupements anhydrides, sulfhydriles ou carboxyles, des groupes dérivés du bromure ou chlorure de cyanogène, du carbonyldiimidazole, des esters de succinimide ou des halogénures sulfoniques.

A cet égard, un objet de l'invention réside également dans un procédé de préparation d'un composé conjugué tel que défini ci-avant, caractérisé en ce qu'il comprend une étape de couplage entre un peptide ou pseudo-peptide V et une substance D, le cas échéant via L, de préférence par voie chimique, biochimique, enzymatique, ou par génie génétique.

Un autre objet de l'invention concerne une composition pharmaceutique caractérisée en ce qu'elle comprend au moins un composé conjugué tel que défini ci-avant et un ou des excipients pharmaceutiquement acceptables.

Un autre objet de l'invention concerne une composition diagnostique caractérisée en ce qu'elle comprend un agent de diagnostic ou d'imagerie médicale constitué d'un composé conjugué tel que défini ci-avant.

Le conjugué peut être utilisé sous forme de tout sel pharmaceutiquement acceptable. Par sels pharmaceutiquement acceptables, on entend par exemple et de manière non limitative des sels d'addition basique ou acide pharmaceutiquement acceptables, hydrates, esters, solvates, précurseurs, métabolites ou stéréoisomères, desdits vecteurs ou conjugués chargés avec au moins une substance d'intérêt.

L'expression sels pharmaceutiquement acceptables se réfère aux sels non toxiques, qui peuvent être généralement préparés en faisant réagir une base libre avec un acide organique ou inorganique convenable. Ces sels conservent l'efficacité biologique et les propriétés des bases libres. Comme exemples représentatifs de tels sels, on peut citer les sels hydrosolubles et hydroinsolubles, tels que les acétates, N-méthylglucamine ammonium, ansonates (4,4-diaminostilbènes-2,2'-disulfonates), benzènesulfonates, benzonates, bicarbonates, bisulfates, bitartrates, borates, bromhydrates, bromures, buryrates, camsylates, carbonates, chlorhydrates, chlorures, citrates, clavulariates, dichlorhydrates, diphosphates, édétates, édétates de calcium, édisylates, estolates, ésylates, fumarates, gluceptates, gluconates, glutamates, glycolylarsanylates, hexafluorophosphates, hexylrésorcinates, hydrabamines, hydroxynaphtoates, iodures, isothionates, lactates, lactobionates, laurates, malates, maléates, mandélates, mésylates, méthylbromures, méthylnitrates, méthylsulfates, mucates, napsylates, nitrates, 3-hydroxy-2-naphtoates, oléates, oxalates, palmitates, pamoates (1,1-méthylène-bis-2-hydroxy-3-naphtoates, ou emboates), pantothénates, phosphates, picrates, polygalacturonates, propionates, p-toluènesulfonates, salicylates, stéarates, subacétates, succinates, sulfates, sulfosalicylates, suramates, tannates, tartrates, téoclates, tosylates, triéthiodides, trifluoroacétates, valérates.

Les compositions de l'invention comprennent avantageusement un véhicule ou excipient pharmaceutiquement acceptable. Le véhicule pharmaceutiquement acceptable peut être choisi parmi les véhicules utilisés de manière classique selon chacun des modes d'administration. En fonction du mode d'administration prévu, les composés peuvent être sous forme solide, semi-solide ou liquide. Pour les compositions solides, telles que comprimés, pilules, poudres ou granulés à l'état libre ou inclus dans des gélules, la substance active peut être combinée avec : a) des diluants, par exemple le lactose, le dextrose, le sucrose, le mannitol, le sorbitol, la cellulose et/ou la glycine ; b) des lubrifiants, par exemple la silice, le talc, l'acide stéarique, son sel de magnésium ou de calcium et/ou le polyéthylène glycol ; c) des liants, par exemple le silicate de magnésium et d'aluminium, la pâte d'amidon, la gélatine, la gomme adragante, la méthylcellulose, la carboxyméthylcellulose sodique et/ou la polyvinylpyrrolidone ; d) des désintégrants, par exemple l'amidon, l'agar, l'acide alginique ou son sel de sodium, ou des mélanges effervescents ; et/ou e) des absorbants, colorants, aromatisants et édulcorants. Les excipients peuvent être par exemple du mannitol, lactose, amidon, stéarate de magnésium, saccharine sodique, talc, cellulose, glucose, sucrose, carbonate de magnésium et analogues de qualité pharmaceutique. Pour les compositions semi-solides, telles que suppositoires, l'excipient peut, par exemple, être une émulsion ou suspension grasse, ou à base de polyalkylène glycol, tel que le polypropylène glycol. Les compositions liquides, en particulier injectables ou à inclure dans une capsule molle, peuvent être préparées par exemple par dissolution, dispersion, etc. de la substance active dans un solvant pharmaceutiquement pur tel que, par exemple, l'eau, le sérum physiologique, le dextrose aqueux, le glycérol, l'éthanol, une huile et ses analogues.

Les compositions ou conjugués de l'invention peuvent être administrés par toute voie adaptée et, de manière non limitative par la voie parentérale, comme par exemple, sous forme de préparations injectables par voie sous-cutanée, intraveineuse ou intramusculaire ; par voie orale (ou *per os*), comme par exemple, sous forme de comprimés enrobés ou non, de gélules, de poudres, de granulés, de suspensions ou de solutions orales (une telle forme pour l'administration par voie orale peut être soit à libération immédiate, soit à libération prolongée ou retardée) ; - voie rectale, comme par exemple, sous forme de suppositoires ; - voie topique, notamment transdermique, comme par exemple, sous la forme de patchs, de pommades ou de gels ; - voie intra-nasale, comme par exemple sous forme d'aérosols et de sprays ; - voie perlinguale ;-voie intraoculaire.

Les compositions pharmaceutiques comprennent typiquement une dose efficace d'un peptide ou pseudo-peptide ou conjugué de l'invention. Une « dose thérapeutiquement efficace » telle qu'elle est décrite ici s'entend comme la dose qui donne un effet thérapeutique pour une condition et un régime d'administration donnés. C'est typiquement la dose moyenne d'une substance active à administrer pour améliorer sensiblement certains des symptômes associés à une maladie ou à un état pathologique. Par exemple, dans le traitement d'un cancer cérébral ou non, d'une pathologie, d'une lésion ou d'un trouble du SNC, la dose d'une substance active qui diminue, empêche, retarde, supprime ou arrête l'une des causes ou l'un des symptômes de la maladie ou du trouble serait thérapeutiquement efficace.

Une « dose thérapeutiquement efficace » d'une substance active n'est pas tenue de guérir une maladie ou un trouble mais fournira un traitement pour cette maladie ou ce trouble de façon à ce que son apparition soit retardée, entravée ou empêchée, ou que ses symptômes soient atténués, ou que son terme soit modifié ou, par exemple, soit moins sévère ou que la récupération du patient soit accélérée.

Il est entendu que la « dose thérapeutiquement efficace » pour une personne en particulier dépendra de divers facteurs, incluant l'activité/efficacité de la substance active, son heure d'administration, sa voie d'administration, son taux d'excrétion et son métabolisme, les associations/interactions médicamenteuses et la sévérité de la maladie (ou du trouble) traitée à titre préventif ou curatif, ainsi que l'âge, le poids corporel, l'état de santé global, le sexe et/ou le régime alimentaire du patient.

Selon la substance couplée, les conjugués et compositions de l'invention peuvent être utilisés pour le traitement, la prévention, le diagnostic ou l'imagerie de nombreuses pathologies, notamment de pathologies affectant le SNC, de pathologies infectieuses ou de cancers.

A cet égard, l'invention concerne l'utilisation de conjugués ou compositions pharmaceutiques tels que décrits ci-avant pour le traitement ou la prévention de pathologies ou troubles du SNC, de tumeurs cancéreuses cérébrales ou d'autres cellules cancéreuses, et de pathologies infectieuses cérébrales ou autres, de type bactériennes, virales, parasitaires ou fongiques.

L'invention concerne également l'utilisation de conjugués ou compositions pharmaceutiques tels que décrits ci-avant pour le diagnostic ou l'imagerie de pathologies ou troubles du SNC, de tumeurs cancéreuses cérébrales ou d'autres cellules cancéreuses, et de pathologies infectieuses cérébrales ou autres, de type bactériennes, virales, parasitaires ou fongiques.

L'invention concerne également l'utilisation d'un conjugué ou d'une composition tels que définis ci-avant pour le traitement, l'imagerie et/ou le diagnostic d'une tumeur cancéreuse cérébrale ou d'un autre type de cellules cancéreuses. Des études ont en effet montré que des patients atteints de certains cancers présentent une hypocholestérolémie. Cette hypocholestérolémie est la conséquence d'une sur-utilisation du cholestérol par les cellules cancéreuses. Ces dernières induisent pour survivre une augmentation du niveau d'expression du LDLR au sein des organes tumoraux (Henricksson et al., 1989, Lancet, 2 (8673), 1178-1180*).* Il existe ainsi une corrélation entre l'augmentation du niveau d'expression du LDLR par les cellules et certains cancers. Il a également été récemment démontré que le nombre de LDLR était très important à la surface de certaines cellules pathologiques comme les cellules cancéreuses. Il est généralement admis que 1 000 à 3 000 LDLR sont présents à la surface d'une cellule non pathologique. De même, des neurones non pathologiques ne présentent qu'un faible nombre de LDLR (Pitas et al., 1987, J. Biol. Chem., 262, 14352-14360*).* Dans le cas du glioblastome, il a été démontré une surexpression du LDLR. Ainsi, il a été dénombré à la surface des cellules cérébrales tumorales de 125 000 (pour les cellules U-251) à 900 000 (pour les cellules SF-767) LDLR (Malentiska et al., 2000, Cancer Res., 60, 2300-2303 *;* Nikanjam et al., 2007, Int. J. Pharm., 328, 86-94*).* Notons également que beaucoup de cellules tumorales surexpriment le LDLR, telles que celles du cancer de la prostate (Chen et al., 2001, Int. J. Cancer, 91, 41-45*),* du cancer du colon (Niendorf et al., 1995, Int. J. Cancer, 61, 461-464*),* des leucémies (Tatidis et al., 2002, Biochem. Pharmacol., 63, 2169-2180)*,* du cancer colorectal (Caruso et al., 2001, Anticancer Res., 21, 429-433), du cancer du sein (Graziani et al., 2002, Gynecol. Oncol., 85, 493-497*),* ainsi que les cancers du foie, du pancréas, des ovaires, du poumon, de l'estomac, etc.

L'invention concerne également l'utilisation d'un conjugué ou d'une composition tels que définis ci-avant pour le traitement, l'imagerie et/ou le diagnostic de pathologies infectieuses cérébrales ou autres, de type bactériennes, virales, parasitaires ou fongiques, telles que, et de manière non limitative, le SIDA, voire les méningites, etc. Le LDLR est également présent sur les cellules hépatiques. Il est à présent connu que l'endocytose du virus de l'hépatite C (VHC) peut se faire par l'intermédiaire du LDLR. Le LDLR pourrait servir de récepteur viral à un stade précoce de l'infection des hépatocytes humains par le VHC (Molina et al., 2007, J. Hepatol., 46 (3), 411-419). Les conjugués de l'invention peuvent donc être utilisés pour cibler spécifiquement des cellules pathologiques, infectées par des virus tels que ceux des hépatites B et C, qui expriment le LDLR et/ou pour moduler le processus d'infection des cellules saines par les virus via le LDLR.

L'invention concerne également l'utilisation d'un conjugué ou d'une composition tels que définis ci-avant pour le traitement, l'imagerie et/ou le diagnostic de pathologies neurodégénératives telles que de manière non limitative la maladie d'Alzheimer, la maladie de Parkinson, la maladie de Creutzfeldt-Jacob, les accidents vasculaires cérébraux (AVC), l'encéphalite bovine spongiforme, la sclérose en plaques, la sclérose latérale amyotrophique, etc.

L'invention concerne également l'utilisation d'un conjugué ou d'une composition tels que définis ci-avant pour le traitement, l'imagerie et/ou le diagnostic de pathologies neurologiques telles que de manière non limitative l'épilepsie, la migraine, les encéphalites, les douleurs du SNC, etc.

L'invention concerne également l'utilisation d'un conjugué ou d'une composition tels que définis ci-avant pour le traitement, l'imagerie et/ou le diagnostic de pathologies neuropsychiatriques telles que de manière non limitative la dépression, l'autisme, l'anxiété, la schizophrénie, etc.

Les termes « traitement », « traiter » et autres expressions semblables signifient l'obtention d'un effet pharmacologique et/ou physiologique, par exemple, l'inhibition de la croissance des cellules cancéreuses, la mort des cellules cancéreuses ou l'amélioration d'une maladie ou d'un trouble neurologique. L'effet peut être prophylactique ou préventif afin d'empêcher totalement ou partiellement l'aggravation d'une maladie ou d'un symptôme de celle-ci, chez une personne malade, ou sa propagation, chez des sujets sains, et/ou peut être thérapeutique afin de traiter totalement ou partiellement une maladie et/ou ses effets néfastes liés. Le terme « traitement » tel qu'il est utilisé dans le présent document couvre tout traitement d'une maladie chez un mammifère, et plus particulièrement un homme, et comprend : (a) la prévention d'une maladie (par exemple, la prévention du cancer) ou d'une condition pouvant survenir chez une personne prédisposée à cette pathologie ou trouble, mais qui n'a pas encore été diagnostiquée comme atteinte, (b) le ralentissement d'une maladie (par exemple, en arrêtant son développement), ou (c) le soulagement d'une maladie (par exemple, en réduisant les symptômes associés à une maladie). Ce terme « traitement » couvre également toute administration d'une substance active afin de soigner, de guérir, de soulager, d'améliorer, de diminuer ou d'inhiber une condition chez un individu ou patient, y compris, sans s'y limiter, l'administration à une personne en ayant besoin d'un médicament composé d'un vecteur ou d'un conjugué tels que décrits dans le présent document.

La présente invention concerne encore l'utilisation d'un peptide ou pseudo-peptide de l'invention pour augmenter l'activité biologique d'une substance active ou d'intérêt (molécule d'intérêt thérapeutique, agent de diagnostic ou d'imagerie médicale, toute autre molécule comme une sonde moléculaire) à laquelle il est couplé.

La présente invention concerne aussi l'utilisation d'un peptide ou pseudo-peptide de l'invention, pour diminuer la toxicité de la substance active ou d'intérêt (molécule d'intérêt thérapeutique, agent de diagnostic ou d'imagerie médicale, toute autre molécule comme une sonde moléculaire) à laquelle il est couplé.

D'autres aspects et avantages de la présente invention apparaîtront à la lecture des exemples ci-dessous, qui sont de nature illustrative seulement mais ne limitent pas la portée de la présente demande.

### EXEMPLES

### EXEMPLE I

### Construction de lignées cellulaires CHO exprimant de manière stable les LDLR humain et murin.

Les inventeurs ont identifié des peptides sur la base de leur interaction et affinité pour les récepteurs humain et murin aux *low-density lipoproteins* (hLDLR et mLDLR), impliqués notamment dans l'endocytose et la transcytose (transport trans-cellulaire, notamment à travers la BHE) du cholestérol. Le préalable à la caractérisation de ces peptides était l'établissement dans des cellules eucaryotes (*Chinese Hamster Ovary Cells,* CHO) de lignées stables exprimant de manière constitutive et à taux élevés le hLDLR et le mLDLR. Ces lignées sont utilisées : i) pour la caractérisation de peptides se fixant sur le hLDLR exprimé à la surface des cellules, dans sa configuration native ; ii) pour vérifier que le hLDLR et le mLDLR peuvent internaliser les peptides sélectionnés par endocytose.

La construction de ces lignées a été décrite dans la demande WO2010/046588. Brièvement, les séquences des ARN messagers codant pour le hLDLR et le mLDLR sont disponibles dans les banques de données (numéros d'accès: NM_000527 et NM_010700 respectivement). Les amorces nécessaires à l'amplification des ADNc par PCR ont été sélectionnées, comportant à leur extrémité (en caractères gras) les sites de restriction nécessaires (HindIII et SalI pour le LDLR humain et HindIII et KpnI pour le LDLR murin) au clonage dans le vecteur d'expression pEGFP-N1 (Clontech) :
hLDLR
   Amorce *Forward*:ATATAT**AAGCTT**CGAGGACACAGCAGGTCGTGAT (SEQ ID NO : 18)
   Amorce *Reverse :* TTAATT**GTCGACC**ACGCCACGTCATCCTCCAGACT (SEQ ID NO : 19)
mLDLR
   Amorce *Forward :* ATATAT**AAGCTT**GACGCAACGCAGAAGCTAAG (SEQ ID NO : 20)
   Amorce *Reverse :* TTAATT**GGTACCG**TTGCCACATCGTCCTCCAG (SEQ ID NO : 21)

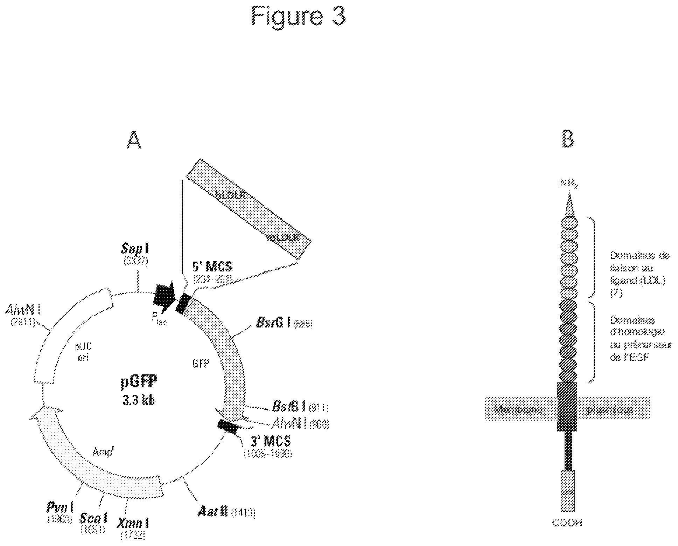

Les ARN totaux préparés à partir de cerveaux humains et murins ont été transformés en ADNc par reverse transcription pour l'amplification par PCR de fragments d'ADN codant pour le hLDLR et le mLDLR. Après amplification, les produits PCR ont été digérés par les enzymes de restriction HindIII-SalI et HindIII-KpnI respectivement et ligués dans le vecteur d'expression pEGFP-N1 (Clontech), digéré par les mêmes enzymes de restriction. Après transfection dans des cellules eucaryotes, ce vecteur permet l'expression, sous contrôle du promoteur CMV, de LDLR fusionnés à la GFP à leur extrémité C-term, c'est à dire à l'extrémité de leurs domaines intracellulaires (Figure 3). Après transformation de bactéries compétentes *E. Coli* DH5α, obtention de colonies isolées, préparation de l'ADN des plasmides, les constructions ont été intégralement séquencées sur les 2 brins pour vérification.

Des transfections transitoires dans différentes lignées cellulaires (CHO, COS, N2A et HEK293) ont été réalisées pour déterminer sur cellules vivantes ou fixées les niveaux d'expression et la localisation membranaire du hLDLR et du mLDLR. Le récepteur est visible directement sur cellules vivantes, sous microscopie à fluorescence, sans nécessité d'immunomarquage, grâce à la fluorescence verte émise par la GFP fusionnée en C-term de ces récepteurs. Des transfectants stables ont été sélectionnés par dilution limite et grâce au gène de résistance à la généticine (G418) porté par le vecteur d'expression. Ces lignées ont été amplifiées tout en gardant une pression de sélection. Dans l'exemple évoqué ici, l'expression du hLDLR-GFP de la taille attendue a été vérifiée par Western Blot sur lysats cellulaires avec des anticorps dirigés contre le LDLR humain et contre la GFP. Une protéine correspondant aux tailles combinées de la GFP et du hLDLR (190 kDa) est reconnue par les anticorps anti-hLDLR (Figure 4) et anti-GFP dans des extraits cellulaires préparés à partir des lignées stables. Une lignée CHO exprimant la GFP de manière constitutive a été utilisée comme contrôle négatif. L'anticorps anti-hLDLR ne détecte aucune protéine dans la lignée GFP.

L'immunocytochimie avec l'anticorps anti-hLDLR sur cellules fixées (PFA) des lignées CHO-GFP (utilisée comme contrôle) et CHO-hLDLR-GFP montre que la fusion hLDLR-GFP est bien exprimée dans les cellules transfectées. Des expériences d'immunocytochimie sur cellules non perméabilisées avec du Triton X100 montrent que le domaine extracellulaire du LDLR est bien détecté au niveau extracellulaire (Figure 5).

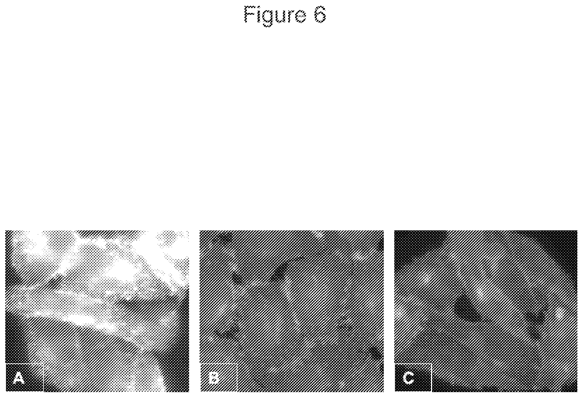

Une co-localisation entre le hLDLR et son ligand naturel le LDL, rendu fluorescent par adsorption du DiI, un marqueur fluorescent, est montrée. Ce ligand naturel fluorescent (DiI-LDL) est rapidement internalisé (endocytose) comme visualisé sous microscopie à fluorescence sur cellules fixées (Figure 6-A). Par contre, le DiI-LDL n'est pas endocyté par la lignée contrôle CHO-GFP, ou par une autre lignée contrôle de cellules CHO, qui surexprime par exemple le récepteur humain de la transferrine (hTfR, Figure 6-B), un autre récepteur impliqué dans la transcytose. De plus, l'activité d'endocytose de la lignée CHO-LDLR-GFP est spécifique au ligand du LDLR puisque dans cette lignée, on n'observe pas d'endocytose de ligand qui ne lui soit pas spécifique, à titre d'exemple la transferrine (Tf) marquée avec un fluorochrome rouge (le *Texas Red,* Figure 6-C).

La fonctionnalité des récepteurs (capacité d'endocytose) est confirmée par des expériences en temps réel, sous vidéo-microscopie à fluorescence montrant que le LDL, ligand naturel du hLDLR, marqué au DiI, est effectivement transporté rapidement et très efficacement dans les cellules exprimant le hLDLR-GFP comparé aux cellules exprimant la GFP seule ou aux cellules exprimant un autre récepteur impliqué dans l'endocytose tel le hTfR (contrôles négatifs). A l'inverse, des expériences de vidéo-microscopie réalisées avec la Tf marquée au *Texas Red,* qui est très efficacement endocytée par des cellules exprimant le hTfR-GFP, confirment que la transferrine n'est pas endocytée par les cellules d'une lignée contrôle GFP ni par celles d'une lignée hLDLR.

Malgré un haut taux d'expression du hLDLR dans les lignées CHO-hLDLR-GFP, le système d'endocytose est non seulement efficace, mais il a conservé sa sélectivité. La présence de la fusion GFP n'a altéré ni les propriétés d'insertion dans la membrane du hLDLR, ni l'exposition à l'extérieur de la cellule du domaine extracellulaire du hLDLR, ni la fonctionnalité du récepteur dans les processus d'endocytose.

### EXEMPLE II

### Synthèses peptidiques et couplages à une molécule traceur (biotine, fluorescéine, ou S-Tag avec une activité enzymatique).

Les peptides ont été synthétisés par la méthode de synthèse en phase solide (*solid phase peptide synthesis* ou SPPS) sur un synthétiseur automatique, modèle Advanced ChemTech Apex396 (AAPPTec), ou un synthétiseur automatique sous champ micro-onde focalisée, modèle Liberty™ (CEM), en utilisant une stratégie Fmoc/tBu sur résine Rink Amide AM sur polystyrène-1%DVB, ou résine Wang sur polystyrène-1%DVB, ou résine Barlos (*2-chlorotrityl chloride*) sur polystyrène-1%DVB, ou résine Sieber Amide sur polystyrène-1%DVB. La charge (ou substitution) est comprise entre 0,25 et 1,6 mmol/g suivant la résine utilisée.

Les acides aminés N-protégés par un Fmoc (ou un Boc pour certaines extrémités N-term) et/ou protégés par des fonctions orthogonales (acido-labiles notamment) au niveau de leurs chaînes latérales, les réactifs chimiques de couplage et de déprotection, et les solvants sont achetés auprès de sociétés spécialisées et utilisés tels quels.

Les résines Rink Amide et Wang permettent de synthétiser les séquences peptidiques totalement déprotégées sur leurs chaînes latérales et leurs extrémités C-term. On parle alors de synthèse peptidique SPPS orthogonale à 2 dimensions (Fmoc/tBu).

Les résines « *hypersensitive acid labile ou HAL »* de type Barlos et Sieber permettent respectivement la libération de la fonction acide ou amide terminale (en C-term) tout en conservant les protections latérales orthogonales des différents acides aminés du peptide synthétisé ainsi que la protection amine terminale (N-term) de la fonction amine de son dernier acide aminé (par exemple, N-acétylation pour des questions de stabilité de la séquence peptidique néo-synthétisée). Ce type de résines via une stratégie de synthèse Fmoc (en Prot₁) permet d'utiliser des protections latérales orthogonales acido-labiles (Prot₂ de type Boc, tBu, OtBu, Trt, Mmt, Acm, etc.) clivables uniquement en milieu fortement acide, alors que le décrochage du peptide protégé se fait en conditions acides très douces. Ce type de clivage permet de récupérer la séquence peptidique totalement protégée sur ses fonctions latérales (Prot₂) en vue notamment du couplage d'une molécule d'intérêt thérapeutique sur le peptide. On parle alors de synthèse peptidique SPPS orthogonale à 3 dimensions (Barlos ou Sieber/Fmoc/tBu).

Les protections latérales orthogonales (Prot₂) standard utilisées pour chaque acide aminé au cours de la synthèse peptidique sont : Arg(N-Pbf), Arg(N-Pmc), Asn(N-Trt), Asp(O-tBu), Cys(S-Acm), Cys(S-Mmt), Cys(S-4MeBn), Cys(S-tBu), Cys(S-Tmob), Cys(S-Trt), Glu(O-tBu), Gln(N-Trt), His(N-Trt), Lys(N-Boc), Pen(S-Acm), Pen(S-Trt), Ser(O-tBu), Thr(O-tBu), Trp(N-Boc), Tyr(O-tBu) (*Applied Biosystems, 1998, Cleavage, Deprotection, and Isolation of Peptides after Fmoc Synthesis. Technical Bulletin).* Gly, Sar, Ala, Val, Leu, Ile, Phe, Met, Pro, Pip et Thz ne possèdent pas de protections latérales, puisque leurs structures chimiques respectives ne le nécessitent pas.

Les couplages d'acides aminés se font via activation de la fonction acide de l'acide aminé n+1 à l'aide de DIEA/HBTU/HOBt ou DIPC/HOBt dans du DMF.

La déprotection du groupement Fmoc (Prot₁) d'un nouvel acide aminé ainsi couplé est réalisée à l'aide de 20% de pipéridine dans le DMF.

Le dernier acide aminé couplé au cours de la séquence peptidique sera soit protégé par une fonction Boc (en vue de libérer sa fonction amine terminale libre en fin de synthèse), soit acétylé (afin de stabiliser le néo-peptide synthétisé mais également de réduire les risques de réactions secondaires lors du couplage covalent de la molécule d'intérêt thérapeutique en C-term par exemple) voire propionylé.

Suivant le peptide synthétisé, les ponts disulfures sont obtenus par cyclisation intramoléculaire à partir de 2 fonctions thiols de 2 Cys convenablement protégées (Acm, Trt, tBu, etc.), soit en solution, soit sur la résine, à l'aide de réactifs classiquement utilisés par l'homme de l'art : H₂O/AcOH/,/(NH₄)₂CO₃/DMSO, H₂O/AcOH,/(NH₄)₂CO₃, I₂/DMF, I₂/HFIP/DCM, TFA/DMSO/anisole, I₂/DCM/MeOH/H₂O, etc. Une Cys en positon N-term peut être avantageusement remplacée par Pen ou Mpa pour la cyclisation via un pont disulfure. Des ponts lanthionines (par cyclisation via déshydroalanine) ou dicarba (par cyclisation via allylGly) peuvent également être obtenus par des voies de synthèse connues de l'homme de l'art. Un pont lactame peut-être créé entre la fonction acide latérale d'un résidu Glu (ou Asp) et une fonction amine latérale sur une Lys ou une amine N-term. De même une cyclistion entre la fonction amine N-term et la fonction acide C-term (tête/queue) peut être réalisée via une liaison amide, tout comme une cyclisation entre la fonction amine latérale d'une Lys et la fonction acide C-term du peptide (Majumdar and Siahaan, Med Res Rev.*, Epub ahead of print*).

Le clivage des peptides des résines Barlos ou Sieber se fait par des méthodes classiquement utilisées par l'homme de l'art, soit avec 0,5% de TFA (v/v) dans du DCM, soit avec de l'AcOH/TFE/DCM (1/1/3), soit avec du HFIP (30%) dans du DCM, soit avec du TFE (30%) dans du DCM, etc.

Les déprotections des chaînes latérales, et le clivage des peptides des résines Rink Amide ou Wang, sont réalisés par des méthodes classiquement utilisées par l'homme de l'art : soit avec un mélange TFA/H₂O/TIS ou TIPS (95/2,5/2,5), soit avec TFA/H₂O/EDT/TIS ou TIPS (94/2,5/2,5/1), soit avec TFA/thioanisole/H₂O (94/5/1), soit avec TFA/TIS/H₂O/thioanisole (90/5/3/2), soit avec TFA/H₂O/phénol/thioanisole/EDT (82,5/5/5/5/2,5), etc.

Les biotines, fluorescéines, ou S-Tag (cf. EXEMPLE IV, ci-dessous) sont généralement introduits en C-term, ces traceurs sont parfois couplés en N-term, selon des procédés de synthèse et de couplage classiques connus de l'homme de l'art.

Les peptides sont isolés et purifiés par HPLC sur un Appareil Beckman System Gold 126 avec une colonne Chromolith C18 (4,6 x 50 mm) ou Nucleosil C18 (10 x 250 mm) avec par exemple un gradient de 0 à 100% d'acétonitrile dans une phase aqueuse (H₂O + 0,1% TFA) en 3,5 min puis 100 à 0% en 1,5 min (débit : 1 à 5 ml/min), ou sur une chaîne Waters 1525 avec une colonne (phase stationnaire) Chromolith Speed ROD RP-18 (4,6 x 50 mm) avec détection par un équipement Waters 996 PDA (190 - 400 nm), ou sur une chaîne Waters Alliance 2690 avec une colonne (phase stationnaire) Chromolith Performance RP-18 (3 x 100 mm) avec détection par un équipement Waters 996 PDA (190 - 400 nm). La détection UV est réalisée à 214 et 254 nm.

Les purifications préparatives sont réalisées avec une chaîne Waters prep LC4000 system avec une colonne (phase stationnaire) Guard-PakTM cartridges Delta-PakTM C18 (25 x 10 mm) avec détection par un équipement Waters 2487 Dual 1 Absorbance Detector.

Les masses moléculaires sont déterminées en utilisant un spectromètre de masse à électrospray d'ions (ESI) en mode positif. Les spectres sont obtenus en utilisant un appareil Waters MicromassQuattro Micro (analyseur quadripôle) équipé d'un système HPLC Waters Alliance 2690 permettant le couplage LC-MS.

Les conditions d'analyses LC-MS utilisées sont les suivantes :
- colonne C18 Chromolith Flash (4,6 x 25 mm),
- debit de 3 mL/min,
- gradient linéaire de 0 à 100 % de B en 2,5 min (A : H2O/HCO2H 0,1 % ; B : ACN/HCO2H 0,1 %).

L'acquisition des spectres de masse en mode electrospray positif se fait à un débit de 100-200 µL/min. Les données sont obtenues en mode scan de 200 à 1700 *m*/*z* à intervalles de 0,1 s.

### EXEMPLE III

### Conception de peptides vecteurs.

Le peptide de séquence SEQ ID NO: 17/S-Tag, décrit dans la demande WO2010/046588, a servi de référence.

A partir de ce peptide, différentes approches ont été utilisées pour tenter d'améliorer les propriétés de liaison, de synthèse et de vectorisation. De nombreux peptides ont ainsi été synthétisés, différant de SEQ ID NO : 17 par la délétion et/ou le remplacement et/ou la modification chimique d'un ou plusieurs résidus d'acide aminé.

La capacité des peptides ainsi synthétisés à lier le hLDLR a été déterminée.

A cet effet, les cellules CHO-hLDLR-RFP adhérentes et à confluence, ont été cultivées dans des plaques 6 puits. Trois puits de cellules sont utilisés par condition.

Une solution contenant 10µM de peptide SEQ ID NO : 17/S-Tag a été préparée dans du milieu de culture HamF12-1% BSA. A cette solution ont été ajoutés 10 µM du peptide à évaluer (compétition).
Plusieurs solutions contrôles sont aussi préparées :
(i) Milieu HamF12-1% BSA.
(ii) Milieu HamF12-1% BSA + 10 µM de peptide contrôle CTRL-S-Tag (évaluation de la liaison non spécifique de tout peptide comportant un S-Tag).
(iii) Milieu HamF12-1% BSA + 10 µM de peptide SEQ ID NO : 17/S-Tag + 10 µM de peptide contrôle CTRL (évaluation de la compétition « non spécifique » entre le peptide d'intérêt et le peptide contrôle CTRL).

Les approches de FRET utilisées sont celles décrites dans l'EXEMPLE IV.

Les résultats obtenus pour les meilleurs peptides sont présentés dans le tableau 1 ci-dessous. Le tableau donne les résultats des compétitions en % de peptide vecteur de référence (SEQ ID NO : 17/S-Tag), affin pour le hLDLR, déplacé par les peptides de l'invention. Plus la valeur de déplacement est grande, plus le peptide est affin pour le hLDLR. Lorsque cette valeur est supérieure à 50%, le peptide possède une affinité supérieure à celle du peptide de référence (SEQ ID NO : 17).

**Tableau 1**

| Peptides tests | Déplacement du peptide contrôle |
|---|---|
| SEQ ID NO : 1 | 66% |
| SEQ ID NO : 2 | 70% |
| SEQ ID NO : 3 | 75% |
| SEQ ID NO : 4 | 88% |
| SEQ ID NO : 5 | 91% |
| SEQ ID NO : 6 | 77% |
| SEQ ID NO : 7 | 74% |
| SEQ ID NO : 8 | 84% |
| SEQ ID NO : 9 | 80% |
| SEQ ID NO : 10 | 54% |
| SEQ ID NO : 11 | 22% |
| SEQ ID NO : 12 | 23% |
| SEQ ID NO : 13 | 8% |
| SEQ ID NO : 14 | 9% |
| SEQ ID NO : 15 | <5% |
| SEQ ID NO : 16 | <5% |
| SEQ ID NO : 17 | 50% |

Les résultats présentés montrent que les peptides de séquences SEQ ID NOs : 1 à 10 présentent une affinité améliorée pour le récepteur humain aux HDL. Ces résultats sont particulièrement intéressants compte tenu de la taille réduite des peptides, et particulièrement remarquables et surprenants, compte tenu des effets négatifs sur l'affinité observés avec les peptides SEQ ID NO : 11-16.

Ainsi, en s'appuyant sur une approche d'optimisation chimique basée sur la chimie médicinale, les inventeurs ont progressé dans la compréhension des relations structure/activité (affinité) du peptide vecteur de référence (SEQ ID NO : 17), et notamment :
(i) l'importance de chacun des résidus d'acides aminés (Ala-scan, D-scan), c'est-à-dire le remplacement alternatif (un par un) de chacun des résidus d'origine soit par une alanine, soit par leur analogue non naturel de configuration D ;
(ii) l'importance des extrémités N- et C-terminales (acétylation, amidation, etc.) ;
(iii) l'importance/intérêt de la cyclisation du peptide.

Les principales limitations généralement attribuées aux dérivés peptidiques à visée thérapeutique sont :
(i) leur faible biodisponibilité orale (leur administration par voie intraveineuse est en général requise) ;
(ii) un temps de demi-vie court en raison de leur dégradation rapide par des enzymes protéolytiques (notamment les peptidases ou protéases) de l'appareil digestif et du plasma sanguin ;
(iii) leur élimination rapide de la circulation sanguine par le foie (clairance hépatique) et les reins (clairance rénale) ;
(iv) leur faible capacité à passer au travers des membranes biologiques ou physiologiques en raison de leur caractère généralement hydrophile ;
(v) leur grande flexibilité conformationnelle, ce qui entraîne parfois un manque de sélectivité impliquant des interactions avec différents récepteurs ou cibles (résultant en une faible biodistribution spécifique), qui a pour effet l'activation de plusieurs cibles et conduit à des effets secondaires ou indésirables ;
(vi) des coûts élevés de synthèse et de production industrielle (le coût de production d'un peptide de masse moléculaire 5 kDa est supérieur au coût de production d'une molécule organique de masse moléculaire 500 Da).

De façon assez remarquable, les peptides ou pseudo-peptides de l'invention présentent les pré-requis pharmacologiques :
(i) une affinité forte pour le hLDLR ;
(ii) des caractéristiques physico-chimiques (cyclisation via un pont disulfure ; jusqu'à 3 acides aminés non naturels pour certains d'entre eux ; une taille réduite à 8 acides aminés) favorisant leur spécificité pour ce récepteur ;
(iii) une plus grande résistance à la protéolyse enzymatique en raison de leur cyclisation, de l'introduction d'acides aminés non naturels et pour certains d'entre eux d'une liaison pseudo-peptidique, mais également en modifiant/bloquant les extrémités N- et C-term ;
(iv) une petite taille et une masse moléculaire voisine de 1 kDa permettant de réduire les coûts de synthèse et de production future à l'échelle industrielle.

### EXEMPLE IV

### Liaison et endocytose des peptides synthétisés, affins pour le hLDLR dans les lignées CHO-LDLR-GFP.

Des peptides de l'invention affins pour hLDLR-GFP sont couplés/conjugués en C-term à différentes molécules « traceurs », soit la rhodamine, soit le S-Tag, séparés par un bras espaceur généralement constitué de 3 résidus Gly (Figure 7). Le S-Tag (peptide de 15 acides aminés dérivé de la séquence 1-15 de la ribonucléase A pancréatique bovine) peut d'une part être reconnu par un anticorps anti-S-Tag pour des approches d'immunocytochimie ou de FACS, et d'autre part reconstituer une activité enzymatique par liaison à la ribonuclease S-protein (portion C-term, acides aminés 21-124) dans des tests *d'activité in vitro* utilisant le FRETWorks S-Tag *assay kit* (Novagen 70724-3). La ribonucléase ainsi activée digère un substrat d'ARN libérant un agent fluorescent masqué révélé par FRET *(fluorescence Resonance Energy Transfert)* et quantifié en plaque de 96 puits dans un spectrofluorimètre Beckmann. Pour ces expériences de FRET, des cellules CHO contrôles ont été utilisées et la GFP fusionnée en C-term au hLDLR et au mLDLR, qui génère un bruit de fond important aux longueurs d'ondes utilisées pour le FRET a été remplacée par la *Red Fluorescent Protein* (RFP). Les lignées stables générées pour les expériences de FRET sont donc CHO-RFP et CHO-hLDLR-RFP.

Pour les approches de FRET, les cellules sont lavées 2 fois avec 2 ml de PBS puis incubées 1h à 37°C avec 250 µl de solution de peptide. Elles sont à nouveau lavées 2 fois avec 2 ml de PBS puis 2 fois avec 1 ml de PBS, puis grattées dans 1 ml de PBS et centrifugées 5 min à 1250 rpm. Le surnageant est alors aspiré et le culot cellulaire est lysé dans 80 µl de PBS + 0.1% de Triton X100. Vingt µl de chaque lysat cellulaire sont analysés par mesure de l'émission de fluorescence après réaction de FRET.

Ainsi, des expériences d'incubation des peptides sur différentes cellules exprimant le hLDLR sont réalisées et permettent de démontrer que les peptides se lient bien aux cellules CHO-LDLR-GFP et qu'ils sont endocytés pour s'accumuler dans les cellules de la lignée qui expriment le hLDLR, ce qui n'est pas le cas des peptides contrôles. Dans ces expériences, des incubations préalables des peptides conjugués au S-Tag, avec un anticorps primaire (Ac primaire) dirigé contre le S-Tag, et un anticorps secondaire (Ac secondaire) dirigé contre l'anticorps primaire, montrent que le complexe entre un peptide/S-Tag, un Ac primaire et un Ac secondaire se lie aux cellules exprimant le hLDLR et est internalisé par endocytose. Ces résultats indiquent que les peptides cycliques de cette famille peuvent se lier à des cellules exprimant le hLDL et vectoriser des charges importantes (2 anticorps), i.e. internalisation par endocytose de ces charges.

### EXEMPLE V

### Toxicité, endocytose et transcytose des peptides synthétisés, affins pour le hLDLR, sur des cellules endothéliales dans des modèles de BHE in vitro.

Les potentiels effets toxiques des peptides sur cellules endothéliales, la liaison/accumulation des peptides dans ces cellules, et le passage par transcytose des peptides sont évalués sur modèles de BHE *in vitro.* Les cellules nécessaires à la mise en place du modèle (co-culture de cellules endothéliales issues de micro-vaisseaux cérébraux et astrocytes) sont des cellules bovines (*bovine brain microvascular endothelial cells,* BBMECs) distribuées par la société Cellial Technologies (Lens, France) ou des cellules de rat (rat brain capillary endothelial cells, rat BCECs) ayant permis de développer en interne un modèle murin. Ce type de modèles de BHE *in vitro* est utilisé pour évaluer le passage passif ou le transport actif de nombreuses molécules, notamment des agents pharmacologiques, à travers les BCECs et donc par extrapolation leur capacité à atteindre le tissu nerveux du SNC *in vivo.* Les modèles bovin et murin présentent des propriétés ultra-structurales caractéristiques de l'endothélium cérébral notamment les jonctions serrées, l'absence de pores, l'absence de canaux trans-endothéliaux, la faible perméabilité aux molécules hydrophiles et une haute résistance électrique. En outre, ces modèles ont montré de bonnes corrélations entre les résultats des mesures effectuées sur différentes molécules évaluées *in vitro* et *in vivo* pour leur propriété de passage au travers de la BHE. A ce jour, toutes les données obtenues révèlent que ces modèles de BHE *in vitro* imitent de près la situation *in vivo* en reproduisant certaines des complexités de l'environnement cellulaire qui existent *in vivo,* tout en conservant les avantages associés à l'expérimentation en culture cellulaire.

A titre d'exemple, le modèle bovin de BHE *in vitro* met en jeu une co-culture de BBMECs et d'astrocytes. Préalablement à la culture cellulaire, des inserts de plaque (Millicell-PC 3,0 µM ; 30 mm de diamètre) sont traités sur la partie supérieure avec du collagène de queue de rat afin de permettre une adhésion optimale des BBMECs et créer les conditions d'une lame basale. Des cultures primaires d'astrocytes mixtes sont établies à partir de cortex cérébral de rat nouveau-né (Dehouck et al., 1990, J. Neurochem., 54, 1798-1801*).* Brièvement, après avoir ôté les méninges, le tissu cérébral est passé sur un tamis de nylon à 82 µm. Les astrocytes sont distribués dans les puits de microplaques à une concentration de 1,2x10⁵ cellules/ml et 2 ml de milieu de culture optimal (DMEM) complémenté avec 10% de sérum de veau foetal inactivé par chauffage. Le milieu est changé deux fois par semaine. Les BBMECs obtenus auprès de Cellial Technologies sont cultivées en présence de milieu DMEM complémenté avec 10% (v/v) de sérum de cheval et 10% de sérum de veau inactivé par chauffage, 2 mM de glutamine, 50 µg/ml de gentamycine, et de 1 ng/ml de facteur de croissance basique de fibroblastes, ajouté tous les deux jours. Les BBMECs sont ensuite distribuées sur la face supérieure des filtres dans 2 ml de la co-culture. Ce milieu de BBMECs est changé trois fois par semaine. Dans ces conditions, des BBMECs différenciées forment une monocouche de cellules confluentes 7 jours plus tard.

Pour tester leur toxicité, les peptides de l'invention couplés à la rhodamine sont incubés dans la chambre supérieure du système de culture, en contact avec les cellules endothéliales pendant 1h, 5h et 24h. Le milieu de culture de la chambre inférieure est collecté à différents temps et la fluorescence quantifiée par analyse fluorimétrique. Les résultats sont exprimés en perméabilité de surface endothéliale (ou Pe) exprimée en 10⁻³ cm/min. Le *Lucifer Yellow* (LY), une petite molécule fluorescente qui passe peu la BHE, est utilisé d'une part pour contrôler l'intégrité de la BHE *in vitro,* dans tous les puits analysés, mais d'autre part, en co-incubation avec les peptides, afin de contrôler l'absence de toxicité des peptides pour les cellules endothéliales qui forment cette BHE. La barrière *in vitro* est considérée « perméable » ou « ouverte » si la valeur de passage Pe du LY est supérieure à 1.10⁻³ cm/min. La mesure de la *Trans Endothelial Electrical Resistence* (TEER), évaluée avec un Ohm-mètre et exprimée en Ohm.cm², permet également de mesurer l'intégrité de la BHE *in vitro* lors des tests de passage à travers cette BHE. La valeur du seuil de qualité est fixée à >500 Ohm.cm².

Les expériences réalisées démontrent une absence de toxicité des peptides, de même que pour le peptide contrôle utilisé, et une absence d'effets délétères sur les propriétés de perméabilité de la BHE.

Le passage à travers la BHE d'un peptide de l'invention conjugué à la rhodamine est vérifié sur le modèle bovin de BHE *in vitro* décrit ci-dessus. Cette analyse est réalisée en mesurant par fluorimétrie la quantité de fluorescence accumulée dans le puits receveur à différents temps (1h, 4h, 24h). L'intégrité de la BHE dans les différents puits analysés est évaluée par mesure simultanée du taux de LY qui passe d'un compartiment à l'autre en fonction du temps.

### EXEMPLE VI

### Protocoles pour la synthèse chimique de conjugués vecteur/molécule d'intérêt thérapeutique ou agent d'imagerie (ou de diagnostic) ou toute autre molécule comme une sonde moléculaire.

Une molécule d'intérêt thérapeutique ou agent d'imagerie ou de diagnostic ou toute autre molécule comme une sonde moléculaire peut être clivée/libérée/relargué(e) du vecteur après transport et passage au travers des membranes cellulaires et plus particulièrement de la BHE, par exemple au travers d'une stratégie prodrogue par hydrolyse ou clivage enzymatique d'une liaison chimique entre le vecteur et la substance active.

Le couplage covalent, entre le peptide vecteur totalement protégé sur ses fonctions réactives latérales (cas des couplages en C-term et N-term) ou partiellement protégé (cas du couplage sur une fonction réactive d'une chaîne latérale), et la molécule d'intérêt thérapeutique, est réalisé via 2 stratégies générales (Figure 2) :
- synthèse en tandem (i.e. couplage direct sans intermédiaire entre les 2 entités),
- synthèse au travers d'un *linker* (Temsamani et al., 2004, Drug Discov. Today, 23, 1012-1019*).*

En fonction du peptide vecteur sélectionné et de la molécule d'intérêt thérapeutique choisie, l'une ou l'autre des diverses stratégies est appliquée soit sur le C-term, soit sur le N-term, soit sur une fonction réactive d'une chaîne latérale de ce peptide vecteur (Majumdar and Siahaan, Med Res Rev.*, Epub ahead of print*)*.* Idéalement, dans une stratégie prodrogue, les bras espaceurs sélectionnés doivent permettre une bonne libération de la substance active et l'amélioration de la solubilité du conjugué (Molema et al., 2001, Drag targeting, organ-specific stratégies. In Methods and principales in medicinal chemistry, vol. 12*).* Diverses liaisons chimiques covalentes labiles peuvent ainsi être générées entre les 2 entités (vecteur et substance active) au travers ou non d'un bras espaceur : amides, carbamates, esters, thioester, disulfure, etc. Par exemple, il a été montré dans la littérature que les liaisons disulfures, relativement stables dans le plasma, peuvent être clivées, par des enzymes telles que la protein-disulfide reductase, à l'intérieur du compartiment intracérébral pour redonner une fonction thiol libre (Saito et al., 2003, Adv. Drug Deliv. Rev., 55, 199-215*).*

D'autres composés intéressants sont ceux dans lesquels le bras espaceur représente un polymère tel que le polyéthylène glycol (PEG). En effet, il a été montré dans la littérature que la conjugaison d'une molécule organique d'intérêt biologique avec un PEG permettait d'augmenter la demi-vie plasmatique de cette molécule *(*Greenwald et al., 2003, Adv. Drug Deliv. Rev., 55, 217-250*)* et de diminuer sa clairance.

Les conjugués vecteurs/substances actives ou d'intérêt sont utilisables en diagnostic, imagerie ou thérapie d'une pathologie, d'une lésion ou d'un trouble du SNC pour la préparation d'un médicament capable de traverser la BHE, d'une tumeur cancéreuse cérébrale ou d'un autre type de cellules cancéreuses pour la préparation d'un médicament capable de traverser les membranes cellulaires cancéreuses, et/ou de pathologies infectieuses pour la préparation d'un médicament capable de traverser les membranes cellulaires et de cibler les cellules infectées des pathologies infectieuses cérébrales ou autres, de type bactériennes, virales, parasitaires ou fongiques.

### EXEMPLE VII

### Perfusion cérébrale in situ pour les vecteurs et les conjugués vecteur/molécule d'intérêt thérapeutique ou agent d'imagerie (ou de diagnostic) ou toute autre molécule comme une sonde moléculaire, et étude de leur cinétique de transport au travers de la BHE, et de leur accumulation dans le cerveau de souris.

La technique de perfusion cérébrale *in situ* (chez la souris mâle adulte OF1) est utilisée pour illustrer le passage dans le cerveau au travers de la BHE.

Au préalable, les peptides vecteurs sont radiomarqués au tritium (³H), élément qui offre la plus forte sensibilité pour la détection de composés radioactifs notamment sur des coupes de tissus. La préparation de peptides radioactifs à haute radioactivité spécifique (RAS, pouvant aller jusqu'à 100 Ci/mmole) est réalisée par une stratégie d'acylation de la fonction amine N-term par de l'anhydride propionique (ou propanoïque) tritié ou du propionyl-N-succinimide (NPS) tritié. Cette méthode de tritiation peut être appliquée à tous les peptides (vecteurs, ou conjugués entre un peptide thérapeutique et un peptide vecteur en tandem ou via un linker (pouvant être de nature peptidique ou organique)), à condition que la modification du N-term n'affecte pas l'affinité des peptides pour le récepteur ciblé (i.e. LDLR) ou leur activité biologique dans le cas de peptides thérapeutiques.
La réaction de tritiation du peptide vecteur en N-term par propionylation est réalisée dans le DMF (1 mg de peptide dans 100 à 450 µl selon solubilité) par ajout de 0,1 équivalent de NPS tritié pendant 5 mn à T ambiante, puis de 0,9 équivalent de NPS froid (non tritié) pendant 1h, puis d'un nouvel équivalent de NPS froid pendant 5 h. Le milieu réactionnel est ensuite laissé à 4°C pendant la nuit et purifié le lendemain par HPLC. La RAS pour chaque peptide tritié est typiquement comprise entre 5 et 10 Ci/mmol. La quantité totale de radioactivité préparée par synthèse est généralement comprise entre 600 et 950 µCi.

Le couplage covalent des peptides radiomarqués (au ³H par exemple) à une substance active radiomarquée (au ¹⁴C par exemple) est effectué, tel que décrit par exemple dans l'EXEMPLE VI. Comme mentionné précédemment, ce couplage covalent est réalisé en fonction de la structure et des propriétés physico-chimiques de la substance active, en particulier la présence de groupes chimiques fonctionnels pouvant être modifiés sans diminuer l'activité biologique de cette substance. Les synthèses des conjugués radiomarqués sont réalisées par extrapolation à partir des voies de synthèse mises au point sur les conjugués non-radiomarqués.

Les techniques brièvement résumées ci-dessous ont été développées précédemment pour étudier la distribution cérébrale de substances actives et en particulier le rôle de la BHE et plus particulièrement du LDLR dans la pénétration de ces molécules dans le cerveau. Les techniques de perfusion cérébrale *in situ* sont parmi les plus exigeantes techniquement et les plus difficiles à réaliser chez la souris. Toutefois, la perfusion cérébrale *in situ* (comme les modèles *in vitro*) permet un contrôle total de la composition du perfusât artificiel, afin de maintenir les cellules et la vascularisation du cerveau dans des conditions physiologiques et anatomiques normales au sein de l'animal, sans le facteur perturbant de distribution systémique.

Cette stratégie de perfusion cérébrale *in situ* normalement effectuée chez le rat a été adaptée chez la souris (Dagenais et al., 2000, J Cereb Blood Flow Metab., 20(2), 381-6) afin d'élargir son application à évaluer les paramètres cinétiques de transport au niveau de la BHE et de la barrière sang-rétine, et ceci également chez des souris transgéniques et KO mutantes pour les récepteurs, enzymes ou transporteurs de substances actives. Il s'agit d'une cathétérisation d'une carotide chez des souris (typiquement OF1) anesthésiées, et de la ligature de certaines branches de cette carotide (externe, thyroïdienne, occipitale) afin de perfuser spécifiquement la carotide interne et les artères ptérygopalatines, qui sont utilisées pour évaluer la captation cérébrale des vecteurs et conjugués. Le cathéter permet de remplacer la circulation générale par une infusion avec un perfusât bien contrôlé (tampon bicarbonate, plasma ou sang) en passant par la carotide. Du tampon bicarbonate de Krebs oxygéné est utilisé en premier lieu afin d'évaluer les capacités de passage cérébral des vecteurs et conjugués. Après cathétérisation de la carotide, le débit sanguin endogène est stoppé en sectionnant les ventricules du coeur afin d'éviter le mélange du tampon avec le sang et l'élévation de la pression sanguine. La durée de la perfusion à débit fixe est contrôlée. La perfusion avec tampon peut être prolongée jusqu'à 20 min, voire jusqu'à 1h en présence de transporteurs d'oxygène (érythrocytes lavés) pour les études de *Receptor-Mediated Transport* (RMT).

Les expériences réalisées ont permis de déterminer le transport cérébral, ou coefficient de transfert (Kᵢₙ : rapport entre le volume de distribution et le temps de perfusion cérébrale), de plusieurs peptides vecteurs de l'invention. Le temps de perfusion cérébrale pour ces expériences est de 5 min avec un débit de perfusât à 2 ml/min. Ainsi, le Kᵢₙ des peptides vecteurs de SEQ ID NO : 2 et SEQ ID NO : 4, radiomarqués à une RAS de 2 Ci/mmole, est respectivement de 3,3 et 3,0 x 10⁻⁴ ml/s/g.

A titre comparatif, la transferrine (Tf) a un Kᵢₙ de 3,0 x 10⁻⁴ ml/s/g (Demeule et al., 2008, J. Neurochem., 106 (4), 1534-1544*),* et le peptide de référence, SEQ ID NO : 17 présente, dans les mêmes conditions expérimentales, un Kᵢₙ de 3,2 ± 0,4 x 10⁻⁴ ml/s/g.

Ces résultats montrent donc que les peptides vecteurs de l'invention, bien que de taille réduite et de configuration avantageuse, possèdent un coefficient de transfert cérébral très élevé, supérieur à celui de la transferrine.

Par ailleurs, ce type d'expérience de perfusion cérébrale *in situ* permet également d'établir une distinction entre les composés qui restent dans le compartiment vasculaire cérébral de ceux ayant franchi la membrane endothéliale abluminale pour entrer dans le parenchyme cérébral. Cette technique, appelée déplétion capillaire post-perfusion, permet de mesurer si la molécule traverse effectivement l'endothélium afin d'entrer dans le parenchyme cérébral. L'utilisation de cette technique permet de démontrer que les peptides vecteurs spécifiques (ou les conjugués) s'accumulent dans le parenchyme cérébral. Ainsi, cette technique (Triguero et al., 1990, J Neurochem., 54(6), 1882-8) est utilisée afin de faire la distinction entre la fraction de vecteurs (ou conjugués), qui a transitée par l'endothélium et est entrée dans le cerveau par l'espace extracellulaire ou les cellules du cerveau, et la fraction restant associée aux cellules endothéliales.

Les étapes clés des études de perfusion cérébrale *in situ* chez la souris peuvent se résumer comme suit pour les vecteurs et conjugués étudiés :
i) Evaluation de la tolérance (non toxicité) des vecteurs et conjugués au niveau de la BHE, conservation de l'intégrité de cette barrière physiologique.
ii) Etude de la cinétique et de la linéarité de la captation cérébrale via RMT par le LDLR.
iii) Etude du taux de captation cérébrale en fonction de la concentration en vecteurs ou en conjugués (Tmax, Km).
iv) Etude des mécanismes de transport à l'aide de substrats inhibiteurs ou modulateurs du LDLR.
v) Distribution dans les compartiments du cerveau : déplétion capillaire (Triguero et al., 1990, J Neurochem., 54(6), 1882-8).
vi) Evaluation du taux de liaison des vecteurs et conjugués aux protéines plasmatiques (albumines, etc.), étude de leur influence sur la captation cérébrale de ces molécules.
vii) Administration intraveineuse et évaluation de la distribution tissulaire (cerveau et autres organes) en fonction du temps.

## Revendications

1. Peptide ou pseudo-peptide, **caractérisé en ce qu'**il répond à la formule générale (I) suivante :
A1-Met-A2-Arg-Leu-Arg-A3-Cys (I)
dans laquelle A1 désigne la cystéine (Cys) ou un de ses analogues ou isostères, A2 désigne la proline (Pro) ou un de ses analogues ou isostères, et A3 désigne la glycine (Gly) ou un des ses analogues ou isostères, étant entendu que le résidu A2 est un analogue de proline lorsque le résidu A1 est une cystéine, et **en ce qu'**il est capable de lier un récepteur humain aux lipoprotéines de faible densité (hLDLR).

2. Peptide ou pseudo-peptide selon la revendication 1, **caractérisé en ce que** A1 désigne Cys ou un de ses analogues choisis parmi la (D)-cys, la Pénicillamine (Pen) et la (D)-pénicillamine ((D)-pen).

3. Peptide ou pseudo-peptide selon la revendication 1 ou 2, **caractérisé en ce que** A2 désigne un analogue de Pro choisi parmi l'acide pipécolique (Pip) et l'acide thiazolidine-4-carboxylique (Thz).

4. Peptide ou pseudo-peptide selon l'une des revendications 1 à 3, **caractérisé en ce que** A3 désigne Gly ou la sarcosine (Sar).

5. Peptide ou pseudo-peptide selon la revendication 1, **caractérisé en ce qu'**il répond à la formule générale (I') suivante :
A1-Met-A2-Arg-Leu-Arg-Gly-Cys (I')
dans laquelle A1 désigne la cystéine ou un de ses analogues ou isostères, de préférence A1 désigne Cys, (D)-cys, Pen, ou (D)-pen ; et A2 désigne Pro ou un de ses analogues ou isostères, de préférence A2 désigne Pip ou Thz, étant entendu que le résidu A2 est un analogue de proline lorsque le résidu A1 est une cystéine et **en ce qu'**il est capable de lier un récepteur humain aux lipoprotéines de faible densité (hLDLR).

6. Peptide ou pseudo-peptide comprenant une séquence choisie parmi séquence SEQ ID NOs : 1 à 10.

7. Peptide ou pseudo-peptide selon l'une quelconque des revendications précédentes, **caractérisé en ce qu'**il est de configuration cyclique.

8. Peptide ou pseudo-peptide selon l'une quelconque des revendications précédentes, **caractérisé en ce qu'**il comprend au moins une liaison peptidomimétique, choisie de préférence parmi l'intercalation d'un groupe méthylène (-CH₂-) ou phosphate (-PO₂-), amine secondaire (-NH-) ou oxygène (-O-), des alpha-azapeptides, alpha-alkylpeptides, N-alkylpeptides, phosphonamidates, depsipeptides, hydroxyméthylènes, hydroxyéthylènes, dihydroxyéthylènes, hydroxyéthylamines, retro-inverso, méthylèneoxy, cétométhylène, esters, phosphinates, phosphiniques, phosphonamides, analogues carba.

9. Peptide ou pseudo-peptide selon l'une quelconque des revendications précédentes, **caractérisé en ce que** la fonction N-terminale (N-term) par une acylation et/ou **en ce que** la fonction C-terminale (C-term) est protégée par une amidation ou une estérification.

10. Peptide ou pseudo-peptide selon l'une quelconque des revendications précédentes, pour son utilisation pour vectoriser une substance active ou d'intérêt en diagnostic, imagerie ou thérapie.

11. Peptide ou pseudo-peptide selon la revendication 10, pour son utilisation pour vectoriser ladite substance dans le système nerveux central.

12. Peptide ou pseudo-peptide selon l'une quelconque des revendications 1 à 9, pour son utilisation pour augmenter l'activité biologique ou diminuer la toxicité d'une substance active ou d'intérêt à laquelle il est couplé.

13. Composé conjugué de formule (III) suivante :
VxLzDy (III)
dans laquelle V représente un peptide ou pseudo-peptide selon l'une quelconque des revendications 1 à 10, L représente un bras espaceur, D représente une substance active ou d'intérêt, x et y sont des nombres entiers compris entre 1 et 5 et z est un entier compris entre 0 et 10.

14. Composé conjugué selon la revendication 13, **caractérisé en ce que** x=z=y=1 ; x=z>y, y=z>x ou z>x>y ; ou **en ce que** z est égal à 0 et x et y sont égal à 1 ou y est supérieur à x.

15. Composé conjugué selon l'une des revendications 13 à 14, **caractérisé en ce que** la substance active ou d'intérêt est une molécule d'intérêt thérapeutique, un agent de diagnostic ou d'imagerie médicale, ou une sonde moléculaire, choisie de préférence parmi une petite molécule chimique, un peptide ou polypeptide, une protéine, un antigène, un anticorps ou une partie d'anticorps, un acide nucléique ou un oligonucléotide, un ribozyme, un marqueur ou un traceur.

16. Composé conjugué selon l'une des revendications 13 à 15, **caractérisé en ce que** le couplage entre V et D, ou entre V et L d'une part et entre L et D d'autre part, est réalisé par une ou plusieurs liaisons covalentes, ioniques, hydrogènes, hydrophobes ou de Van der Waals, clivables ou non en milieu physiologique ou à l'intérieur de cellules.

17. Composé conjugué selon l'une quelconque des revendications 13 à 16, **caractérisé en ce que** D est couplé à V, le cas échéant via L, au niveau d'une ou des extrémités N-term et/ou C-term de V et/ou au niveau d'un ou plusieurs groupements réactifs portés par les chaînes latérales des acides aminés naturels ou non, constitutifs de V.

18. Procédé de préparation d'un composé conjugué selon l'une des revendications 13 à 17, **caractérisé en ce qu'**il comprend une étape de couplage entre un peptide ou pseudo-peptide V et une substance D, le cas échéant via L, de préférence par voie chimique, biochimique, enzymatique, ou par génie génétique.

19. Composition pharmaceutique **caractérisée en ce qu'**elle comprend au moins un composé conjugué selon l'une des revendications 13 à 18 et un ou des excipients pharmaceutiquement acceptables.

20. Composition diagnostique **caractérisée en ce qu'**elle comprend un agent de diagnostic ou d'imagerie médicale constitué d'un composé conjugué selon l'une des revendications 13 à 18.

## Patentansprüche

1. Peptid oder Pseudopeptid, **dadurch gekennzeichnet, dass** es der folgenden allgemeinen Formel (I) entspricht:
A1-Met-A2-Arg-Leu-Arg-A3-Cys (I)
in der A1 für Cystein (Cys) oder eines seiner Analoga oder Isostere steht, A2 für Prolin (Pro) oder eines seiner Analoga oder Isostere steht und A3 für Glycin (Gly) oder eines seiner Analoga oder Isostere steht, unter der Voraussetzung, dass der Rest A2 ein Analogon von Prolin ist, wenn der Rest A1 ein Cystein ist, und **dadurch gekennzeichnet, dass** es in der Lage ist, einen humanen Rezeptor für Lipoproteine geringer Dichte (hLDLR) zu binden.

2. Peptid oder Pseudopeptid gemäß Anspruch 1, **dadurch gekennzeichnet, dass** A1 für Cys oder eines seiner Analoga steht, ausgewählt aus (D)-Cys, Penicillamin (Pen) und (D)-Penicillamin ((D)-Pen).

3. Peptid oder Pseudopeptid gemäß Anspruch 1 oder 2, **dadurch gekennzeichnet, dass** A2 für ein Analogon von Pro steht, ausgewählt aus Pipecolinsäure (Pip) und Thiazolidin-4-carbonsäure (Thz).

4. Peptid oder Pseudopeptid gemäß einem der Ansprüche 1 bis 3, **dadurch gekennzeichnet, dass** A3 für Gly oder Sarcosin (Sar) steht.

5. Peptid oder Pseudopeptid gemäß Anspruch 1, **dadurch gekennzeichnet, dass** es der folgenden allgemeinen Formel (I') entspricht:
A1-Met-A2-Arg-Leu-Arg-Gly-Cys (I')
in der A1 für Cystein (Cys) oder eines seiner Analoga oder Isostere steht, vorzugsweise A1 für Cys, (D)-Cys, Pen oder (D)-Pen steht; und A2 für Pro oder eines seiner Analoga oder Isostere steht, vorzugsweise A2 für Pip oder Thz steht, unter der Voraussetzung, dass der Rest A2 ein Analogon von Prolin ist, wenn der Rest A1 ein Cystein ist, und **dadurch gekennzeichnet, dass** es in der Lage ist, einen humanen Rezeptor für Lipoproteine geringer Dichte (hLDLR) zu binden.

6. Peptid oder Pseudopeptid, umfassend eine Sequenz, ausgewählt aus Sequenz SEQ ID NR: 1 bis 10.

7. Peptid oder Pseudopeptid gemäß einem der vorhergehenden Ansprüche, **dadurch gekennzeichnet, dass** es eine cyclische Konfiguration besitzt.

8. Peptid oder Pseudopeptid gemäß einem der vorhergehenden Ansprüche, **dadurch gekennzeichnet, dass** es wenigstens eine peptidomimetische Bindung umfasst, bevorzugt ausgewählt aus der Interkalation von einer Methylen- (-CH₂-) oder Phosphatgruppe (-PO₂-), sekundärem Amin (-NH-) oder Sauerstoff (-O-), alpha-Azapeptiden, alpha-Alkylpeptiden, N-Alkylpeptiden, Phosphonamidaten, Depsipeptiden, Hydroxymethylenen, Hydroxyethylenen, Dihydroxyethylenen, Hydroxyethylaminen, retro-inverso, Methylenoxy, Ketomethylen, Estern, Phosphinaten, Phosphinsäuren, Phosphonamiden, Carba-Analoga.

9. Peptid oder Pseudopeptid gemäß einem der vorhergehenden Ansprüche, **dadurch gekennzeichnet, dass** die N-terminale (N-term) Funktion durch eine Acylierung geschützt ist und/oder **dadurch gekennzeichnet, dass** die C-terminale (C-term) Funktion durch eine Amidierung oder eine Veresterung geschützt ist.

10. Peptid oder Pseudopeptid gemäß einem der vorhergehenden Ansprüche zur Verwendung für eine Vektorisierung einer aktiven Substanz oder einer Substanz von Interesse in Diagnose, Bildgebung oder Therapie.

11. Peptid oder Pseudopeptid gemäß Anspruch 10 zur Verwendung für eine Vektorisierung besagter Substanz im Zentralnervensystem.

12. Peptid oder Pseudopeptid gemäß einem der Ansprüche 1 bis 9 zur Verwendung für eine Erhöhung der biologischen Aktivität oder Verringerung der Toxizität einer aktiven Substanz oder Substanz von Interesse, an der es gekoppelt ist.

13. Konjugierte Verbindung der folgenden Formel (III):
VxLzDy (III)
in der V für ein Peptid oder Pseudopeptid gemäß einem der Ansprüche 1 bis 10 steht, L für einen Spacer steht, D für eine aktive Substanz oder eine Substanz von Interesse steht, x und y ganze Zahlen zwischen 1 und 5 sind und z eine ganze Zahl zwischen 0 und 10 ist.

14. Konjugierte Verbindung gemäß Anspruch 13, **dadurch gekennzeichnet, dass** x=z=y=1; x=z>y, y=z>x oder z>x>y, oder **dadurch gekennzeichnet, dass** z gleich 0 ist und x und y gleich 1 sind oder y größer als x ist.

15. Konjugierte Verbindung gemäß einem der Ansprüche 13 bis 14, **dadurch gekennzeichnet, dass** die aktive Substanz oder die Substanz von Interesse ein Molekül von therapeutischem Interesse, ein Agens für Diagnose oder medizinische Bildgebung oder eine molekulare Sonde ist, ausgewählt bevorzugt aus einem kleinen chemischen Molekül, einem Peptid oder Polypeptid, einem Protein, einem Antigen, einem Antikörper oder einem Teil eines Antikörpers, einer Nukleinsäure oder einem Oligonukleotid, einem Ribozym, einem Marker oder einem Tracer.

16. Konjugierte Verbindung gemäß einem der Ansprüche 13 bis 15, **dadurch gekennzeichnet, dass** die Kopplung zwischen V und D oder zwischen V und L einerseits und zwischen L und D andererseits durch eine oder mehrere kovalente, ionische, hydrophobe, Wasserstoffbrücken- oder van-der-Waals-Bindungen erfolgt, die in physiologischem Medium oder im Zellinnern gespalten werden können.

17. Konjugierte Verbindung gemäß einem der Ansprüche 13 bis 16, **dadurch gekennzeichnet, dass** D an V, gegebenenfalls über L, an einem oder beiden N-term- und/oder C-term-Enden von V und/oder an einer oder mehreren reaktiven Gruppen in den Seitenketten der natürlichen oder nichtnatürlichen Aminosäuren, die für V konstitutiv sind, gekoppelt ist.

18. Verfahren zur Herstellung einer konjugierten Verbindung gemäß einem der Ansprüche 13 bis 17, **dadurch gekennzeichnet, dass** es einen Kopplungsschritt zwischen einem Peptid oder Pseudopeptid V und einer Substanz D, gegebenenfalls über L, bevorzugt auf chemische, biochemische, enzymatische Weise oder mittels Gentechnik umfasst.

19. Pharmazeutische Zusammensetzung, **dadurch gekennzeichnet, dass** sie wenigstens eine konjugierte Verbindung gemäß einem der Ansprüche 13 bis 18 und einen oder mehrere pharmazeutisch verträgliche Träger umfasst.

20. Diagnostische Zusammensetzung, **dadurch gekennzeichnet, dass** sie ein Agens für Diagnose oder medizinische Bildgebung umfasst, das aus einer konjugierten Verbindung gemäß einem der Ansprüche 13 bis 18 besteht.

## Claims

1. A peptide or pseudo-peptide, **characterized in that** it is of the following general formula (I):
A1-Met-A2-Arg-Leu-Arg-A3-Cys (I)
wherein A1 represents cysteine (Cys) or an analogue or isostere thereof, A2 represents proline (Pro) or an analogue or isostere thereof, and A3 represents glycine (Gly) or an analogue or isostere thereof, residue A2 being a proline analogue when residue A1 is a cysteine, and **in that** it is able to bind a human low-density lipoprotein receptor (hLDLR).

2. The peptide or pseudo-peptide of claim 1, **characterized in that** A1 represents Cys or an analogue thereof selected from (D)-cys, penicillamine (Pen) and (D)-penicillamine ((D)-Pen).

3. The peptide or pseudo-peptide of claim 1 or 2, **characterized in that** A2 represents a Pro analogue selected from pipecolic acid (Pip) and thiazolidine-4-carboxylic acid (Thz).

4. The peptide or pseudo-peptide of any one of claims 1 to 3, **characterized in that** A3 represents Gly or sarcosin (Sar).

5. The peptide or pseudo-peptide of claim 1, **characterized in that** it is of the following general formula (I'):
A1- Met-A2-Arg-Leu-Arg-Gly-Cys (I')
wherein A1 represents cysteine or an analogue or isostere thereof, preferably A1 represents Cys, (D)-cys, Pen, or (D)-Pen; and A2 represents Pro or an analogue or isostere thereof, preferably A2 represents Pip or Thz, residue A2 being a proline analogue when residue A1 is a cysteine, and **in that** it is able to bind a human low-density lipoprotein receptor (hLDLR).

6. A peptide or pseudo-peptide comprising a sequence selected from SEQ ID NOs: 1 to 10.

7. The peptide or pseudo-peptide of any of the preceding claims, **characterized in that** it has a cyclic configuration.

8. The peptide or pseudo-peptide of any of the preceding claims, **characterized in that** it contains at least one peptidomimetic bond, chosen preferably from intercalation of a methylene (-CH₂-) or phosphate (-PO₂-) group, secondary amine (-NH-) or oxygen (-O-), alpha-azapeptides, alpha-alkylpeptides, N-alkylpeptides, phosphonamidates, depsipeptides, hydroxymethylenes, hydroxyethylenes, dihydroxyethylenes, hydroxyethylamines, retro-inverso, methyleneoxy, cetomethylene, esters, phosphinates, phosphinics, phosphonamides and carba analogues.

9. The peptide or pseudo-peptide of any of the preceding claims, **characterized in that** the N-terminal (N-term) function is protected by acylation and/or **in that** the C-terminal (C-term) function is protected by amidation or esterification.

10. The peptide or pseudo-peptide of any of the preceding claims, for use to vectorize an active substance or a substance of interest in diagnostics, imaging or therapy.

11. The peptide or pseudo-peptide of claim 10, for use to vectorize said substance in the central nervous system.

12. The peptide or pseudo-peptide of anyone of claims 1 to 9, for use to increase the biological activity or to decrease the toxicity of an active substance or of a substance of interest to which it is coupled.

13. A conjugated compound of following formula (III):
VxLzDy (III)
wherein V represents a peptide or pseudo-peptide according to any one of claims 1 to 10, L represents a spacer, D represents an active substance or a substance of interest, x and y are integers between 1 and 5 and z is a integer between 0 and 10.

14. The conjugated compound of claim 13, **characterized in that** x=z=y=1; x=z>y, y=z>x or z>x>y; or **in that** z is equal to 0 and x and y are equal to 1 or y is greater than x.

15. The conjugated compound of claim 13 or 14, **characterized in that** the active substance or substance of interest is a molecule of therapeutic interest, a diagnostic or medical imaging agent, or a molecular probe, preferably selected among a small chemical molecule, a peptide or polypeptide, a protein, an antigen, an antibody or part of an antibody, a nucleic acid or an oligonucleotide, a ribozyme, a marker or a tracer.

16. The conjugated compound of any one of claims 13 to 15, **characterized in that** the coupling between V and D, or between V and L on the one hand and L and D on the other, is carried out by one or more covalent, ionic, hydrogen, hydrophobic or Van der Waals bonds, cleavable or not in physiological medium or within cells.

17. The conjugated compound of any one of claims 13 to 16, **characterized in that** D is coupled with V, if necessary via L, at one or both of the N-term and/or C-term ends of V and/or at one or more reactive groups carried by the side chains of the natural or non-natural amino acids constitutive of V.

18. A method for preparing a conjugated compound of any one of claims 13 to 17, **characterized in that** it comprises a coupling step between a peptide or pseudo-peptide V and a substance D, if necessary via L, preferably by chemical, biochemical or enzymatic route or by genetic engineering.

19. A pharmaceutical composition **characterized in that** it comprises at least one conjugated compound of any one of claims 13 to 18 and one or several pharmaceutically acceptable excipients.

20. A diagnostic composition **characterized in that** it comprises a diagnostic or a medical imaging agent consisting of a conjugated compound of any one of claims 13 to 18.


### SEQUENCE LISTING

<110> VECT-HORUS
   CNRS
   UNIVERSITE MEDITERRANEE
<120> DERIVES **PEPTIDIQUES,** LEUR PREPARATION ET LEURS UTILISATIONS
<130> B1026
<160> 21
<170> PatentIn version 3.3
<210> 1
   <211> 8
   <212> PRT
   <213> Artificial
<220>
   <223> peptide
<220>
   <221> MISC_FEATURE
   <222> (1)..(1)
   <223> Xaa = (D)-cys
<220>
   <221> MISC_FEATURE
   <222> (3)..(3)
   <223> Xaa = Pip
<400> 1
   Xaa Met Xaa Arg Leu Arg Gly Cys
   1               5
<210> 2
   <211> 8
   <212> PRT
   <213> Artificial
<220>
   <223> peptide
<220>
   <221> MISC_FEATURE
   <222> (1)..(1)
   <223> Xaa = (D)-pen
<220>
   <221> MISC_FEATURE
   <222> (3)..(3)
   <223> Xaa = Pip
<400> 2
   Xaa Met Xaa Arg Leu Arg Gly Cys
   1               5
<210> 3
   <211> 8
   <212> PRT
   <213> Artificial
<220>
   <223> peptide
<220>
   <221> MISC_FEATURE
   <222> (1)..(1)
   <223> Xaa = Pen
<220>
   <221> MISC_FEATURE
   <222> (3)..(3)
   <223> Xaa = Pip
<400> 3
   Xaa Met Xaa Arg Leu Arg Gly Cys
   1               5
<210> 4
   <211> 8
   <212> PRT
   <213> Artificial
<220>
   <223> peptide
<220>
   <221> MISC_FEATURE
   <222> (1)..(1)
   <223> Xaa = (D)-cys
<220>
   <221> MISC_FEATURE
   <222> (3)..(3)
   <223> Xaa = Thz
<400> 4
   Xaa Met Xaa Arg Leu Arg Gly Cys
   1 5
<210> 5
   <211> 8
   <212> PRT
   <213> Artificial
<220>
   <223> peptide
<220>
   <221> MISC_FEATURE
   <222> (1)..(1)
   <223> Xaa = (D)-pen
<220>
   <221> MISC_FEATURE
   <222> (3)..(3)
   <223> Xaa = Thz
<400> 5
   Xaa Met Xaa Arg Leu Arg Gly Cys
   1 5
<210> 6
   <211> 8
   <212> PRT
   <213> Artificial
<220>
   <223> peptide
<220>
   <221> MISC_FEATURE
   <222> (1)..(1)
   <223> Xaa = Pen
<220>
   <221> MISC_FEATURE
   <222> (3) .. (3)
   <223> Xaa = Thz
<400> 6
   Xaa Met Xaa Arg Leu Arg Gly Cys
   1               5
<210> 7
   <211> 8
   <212> PRT
   <213> Artificial
<220>
   <223> peptide
<220>
   <221> MISC_FEATURE
   <222> (1)..(1)
   <223> Xaa = (D)-cys
<220>
   <221> MISC_FEATURE
   <222> (3)..(3)
   <223> Xaa = Thz
<220>
   <221> MISC_FEATURE
   <222> (7).. (7)
   <223> Xaa = Sar
<400> 7
   Xaa Met Xaa Arg Leu Arg Xaa Cys
   1               5
<210> 8
   <211> 8
   <212> PRT
   <213> Artificial
<220>
   <223> peptide
<220>
   <221> MISC_FEATURE
   <222> (1)..(1)
   <223> Xaa = (D)-pen
<220>
   <221> MISC_FEATURE
   <222> (3)..(3)
   <223> Xaa = Thz
<220>
   <221> MISC_FEATURE
   <222> (7)..(7)
   <223> Xaa = Sar
<400> 8
   Xaa Met Xaa Arg Leu Arg Xaa Cys
   1               5
<210> 9
   <211> 8
   <212> PRT
   <213> Artificial
<220>
   <223> peptide
<220>
   <221> MISC_FEATURE
   <222> (1)..(1)
   <223> Xaa = Pen
<220>
   <221> MISC_FEATURE
   <222> (3)..(3)
   <223> Xaa = Thz
<220>
   <221> MISC_FEATURE
   <222> (7)..(7)
   <223> Xaa = Sar
<400> 9
   Xaa Met Xaa Arg Leu Arg Xaa Cys
   1               5
<210> 10
   <211> 8
   <212> PRT
   <213> artificial
<220>
   <223> peptide
<220>
   <221> MISC_FEATURE
   <222> (1)..(1)
   <223> Cys at position 1 is in configuration D
<220>
   <221> MISC_FEATURE
   <222> (7)..(7)
   <223> Ala at position 7 is in configuration D
<400> 10
   Cys Met Pro Arg Leu Arg Ala Cys
   1               5
<210> 11
   <211> 8
   <212> PRT
   <213> Artificial
<220>
   <223> peptide
<220>
   <221> MISC_FEATURE
   <222> (1)..(1)
   <223> Xaa = (D)-cys
<220>
   <221> MISC_FEATURE
   <222> (4)..(4)
   <223> Xaa = hArg
<400> 11
   Xaa Met Pro Xaa Leu Arg Gly Cys
   1               5
<210> 12
   <211> 8
   <212> PRT
   <213> Artificial
<220>
   <223> peptide
<220>
   <221> MISC_FEATURE
   <222> (1)..(1)
   <223> Xaa = (D)-cys
<220>
   <221> MISC_FEATURE
   <222> (4)..(4)
   <223> Xaa = Agb
<400> 12
   Xaa Met Pro Xaa Leu Arg Gly Cys
   1               5
<210> 13
   <211> 8
   <212> PRT
   <213> Artificial
<220>
   <223> peptide
<220>
   <221> MISC_FEATURE
   <222> (1)..(1)
   <223> Xaa = (D)-cys
<220>
   <221> MISC_FEATURE
   <222> (4)..(4)
   <223> Xaa = Agp
<400> 13
   Xaa Met Pro Xaa Leu Arg Gly Cys
   1               5
<210> 14
   <211> 8
   <212> PRT
   <213> Artificial
<220>
   <223> peptide
<220>
   <221> MISC_FEATURE
   <222> (1)..(1)
   <223> Xaa = (D)-cys
<220>
   <221> MISC_FEATURE
   <222> (4)..(4)
   <223> Xaa = Cit
<400> 14
   Xaa Met Pro Xaa Leu Arg Gly Cys
   1               5
<210> 15
   <211> 8
   <212> PRT
   <213> Artificial
<220>
   <223> peptide
<220>
   <221> MISC_FEATURE
   <222> (1)..(1)
   <223> Xaa = (D)-cys
<220>
   <221> MISC_FEATURE
   <222> (6)..(6)
   <223> Xaa = Cit
<400> 15
   Xaa Met Pro Arg Leu Xaa Gly Cys
   1 5
<210> 16
   <211> 8
   <212> PRT
   <213> Artificial
<220>
   <223> peptide
<220>
   <221> MISC_FEATURE
   <222> (1)..(1)
   <223> Xaa = (D)-cys
<220>
   <221> MISC_FEATURE
   <222> (6)..(6)
   <223> Xaa = Arg(NO2)
<400> 16
   Xaa Met Pro Arg Leu Xaa Gly Cys
   1 5
<210> 17
   <211> 8
   <212> PRT
   <213> Artificial
<220>
   <223> peptide
<220>
   <221> MISC_FEATURE
   <222> (1)..(1)
   <223> Xaa = (D)-cys
<400> 17
   Xaa Met Pro Arg Leu Arg Gly Cys
   1 5
<210> 18
   <211> 34
   <212> DNA
   <213> Artificial
<220>
   <223> primer
<400> 18
   atatataagc ttcgaggaca cagcaggtcg tgat 34
<210> 19
   <211> 35
   <212> DNA
   <213> Artificial
<220>
   <223> primer
<400> 19
   ttaattgtcg accacgccac gtcatcctcc agact 35
<210> 20
   <211> 32
   <212> DNA
   <213> Artificial
<220>
   <223> primer
<400> 20
   atatataagc ttgacgcaac gcagaagcta ag 32
<210> 21
   <211> 32
   <212> DNA
   <213> Artificial
<220>
   <223> primer
<400> 21
   ttaattggta ccgttgccac atcgtcctcc ag 32